# EUROPEAN PATENT SPECIFICATION

(11) **EP 1 663 194 B1**
(45) Date of publication and mention of the grant of the patent: **31.03.2010**
(21) Application number: 04782425.5
(22) Date of filing: 26.08.2004
(51) Int. Cl.: A61K 31/185, A61P 35/00, A61P 43/00, A61K 31/16

(54) **Use of SAHA for treating mesothelioma**
Verwendung von SAHA zur Behandlung von Mesotheliom
Utilisation de SAHA pour traiter le mésotheliome

(30) Priority: 26.08.2003 US 650025; 16.09.2003 US 665079
(43) Date of publication of application: 07.06.2006
(73) Proprietor: Merck HDAC Research, LLC, Boston, MA 02115 (US); Sloan-Kettering Institute for Cancer Research, New York, NY 10021 (US)
(72) Inventor: Bacopoulos, Nicholas, G., New York, NY 10023 (US); CHIAO, Judy, H., Berkeley Heights, NJ 07922 (US); MILLER, Thomas, A., Brookline MA 02445 (US); PARADISE, Carolyn, M., Cortland Manor, NY 10567 (US); RICHON, Victoria, M., Wellesley MA 02481 (US)
(74) Representative: Crump, Julian Richard John
(86) International application number: PCT/US2004/027943
(87) International publication number: WO 2005/018578

(56) References cited:
- WO-A-03/075839
- WO-A-2004/096224
- WO-A1-03/013493
- US-A1- 2003 161 830
- US-A1- 2004 127 523
- ZHANG C ET AL: "The histone deacetylase inhibitor suberoylanilide hydroxamic acid induces apoptosis in cutaneous T cell lymphoma cells." JOURNAL OF INVESTIGATIVE DERMATOLOGY, vol. 121, no. 1, July 2003 (2003-07), page 1189, XP002445157 & INTERNATIONAL INVESTIGATIVE DERMATOLOGY 2003 : JOINT MEETING OF THE EUROPEAN SOCIETY FOR DERMATOLOGI; MIAMI BEACH, FLORIDA, USA; APRIL 30-MAY 04, 2003 ISSN: 0022-202X
- O'CONNOR OWEN A ET AL: "Clinical Experience of the Histone Deacetylase Inhibitor Suberoylanalide Hydroxamic Acid (SAHA) in Heavily Pre-Treated Patients with Aggressive Non-Hodgkin's Lymphoma (NHL0 and Hodgkin's Disease (HD)." BLOOD, vol. 100, no. 11, 16 November 2002 (2002-11-16), page Abstract No. 4742, XP009087702 & 44TH ANNUAL MEETING OF THE AMERICAN SOCIETY OF HEMATOLOGY; PHILADELPHIA, PA, USA; DECEMBER 06-10, 2002 ISSN: 0006-4971
- KELLY W. ET AL.: "A phase I clinical trial of an oral formulation of the histone deacetylase inhibitor suberoylanilide hydroxamic acid (SAHA)" EUROPEAN JOURNAL OF CANCER, PERGAMON PRESS, OXFORD, GB, vol. 38, November 2002 (2002-11), page S88, XP004403728 ISSN: 0959-8049
- DATABASE INTERNET [Online] Proc. Am. Soc. Clin. Oncol. (abstr. 2321) 3 June 2003 (2003-06-03), HEANEY M. ET AL.: "Clinical experience with the histone deacetylase (HDAC) inhibitor suberoylanilide hydroxamic acid (SAHA) in heavily pre-treated patients with hematological malignancies." XP007902727 retrieved from INTERNET accession no. http://www.asco.org/
- DATABASE BIOSIS [Online] BIOSCIENCES INFORMATION SERVICE, PHILADELPHIA, PA, US; 16 November 2001 (2001-11-16), O'CONNOR OWEN A ET AL: "Clinical development of the histone deacetylase inhibitor suberoylanalide hydroxamic acid (SAHA) in aggressive non-Hodgkin's lymphoma (NHL) and Hodgkin's disease (HD)" XP002445158 Database accession no. PREV200200220180 & BLOOD, vol. 98, no. 11 Part 1, 16 November 2001 (2001-11-16), page 611a, 43RD ANNUAL MEETING OF THE AMERICAN SOCIETY OF HEMATOLOGY, PART 1; ORLANDO, FLORIDA, USA; DECEMBER 07-11, 2001 ISSN: 0006-4971
- KELLY W K ET AL: "HISTONE DEACETYLASE INHIBITORS: FROM TARGET TO CLINICAL TRIALS" EXPERT OPINION ON INVESTIGATIONAL DRUGS, ASHLEY PUBLICATIONS LTD., LONDON, GB, vol. 11, no. 12, 1 December 2002 (2002-12-01), pages 1695-1713, XP001202636 ISSN: 1354-3784
- CAO XIAOBO X ET AL: "Histone deacetylase inhibitor downregulation of bcl-xl gene expression leads to apoptotic cell death in mesothelioma" AMERICAN JOURNAL OF RESPIRATORY CELL AND MOLECULAR BIOLOGY, vol. 25, no. 5, November 2001 (2001-11), pages 562-568, XP007902735 ISSN: 1044-1549
- RICHON V M: "Cancer biology: mechanism of antitumour action of vorinostat (suberoylanilide hydroxamic acid), a novel histone deacetylase inhibitor" BRITISH JOURNAL OF CANCER, LONDON, GB, vol. 95, no. suppl, 2006, pages s2-s6, XP007902719 ISSN: 0007-0920
- DATABASE CAPLUS [Online] WU ET AL: 'Negative regulation of bcl-2 expression by p53 in hematopoietic cells', XP002986012 Retrieved from STN Database accession no. (2001:114006) & ONCOGENE vol. 20, no. 2, 2001, pages 240 - 251

## Description

### FIELD OF THE INVENTION

The present invention relates to pharmaceutical compositions for use in methods of treating cancers, e.g., mesothelioma. More specifically, the present invention relates to the use of pharmaceutical compositions comprising suberoylanilide hydroxamic acid (SAHA) for the preparation of a medicament for the treatment of mesothelioma. The oral formulations of the pharmaceutical compositions have favorable pharmacokinetic profiles such as high bioavailability and surprisingly give rise to high blood levels of the active compounds over an extended period of time.

### BACKGROUND OF THE INVENTION

Throughout this application various publications are referenced by arabic numerals within parentheses. Full citations for these publications may be found at the end of the specification immediately preceding the claims.

Cancer is a disorder in which a population of cells has become, in varying degrees, unresponsive to the control mechanisms that normally govern proliferation and differentiation.

Mesothelioma is a rare form of cancer in which malignant (cancerous) cells are found in the mesothelium, a protective sac that covers most of the body's internal organs. The mesothelium is a membrane that covers and protects most of the internal organs of the body. It is composed of two layers of cells: one layer immediately surrounds the organ; the other forms a sac around it. The mesothelium produces a lubricating fluid that is released between these layers, allowing moving organs (such as the beating heart and the expanding and contracting lungs) to glide easily against adjacent structures.

The mesothelium has different names, depending on its location in the body. The peritoneum is the mesothelial tissue that covers most of the organs in the abdominal cavity. The pleura is the membrane that surrounds the lungs and lines the wall of the chest cavity. The pericardium covers and protects the heart. The mesothelial tissue surrounding the male internal reproductive organs is called the tunica vaginalis testis. The tunica serosa uteri covers the internal reproductive organs in women. Most cases of mesothelioma begin in the pleura or peritoneum. Malignant tumors arising from the pleural mesothelium are strongly linked to asbestos exposure.

Although reported incidence rates have increased in the past 20 years, mesothelioma is still a relatively rare cancer. About 2,000 new cases of mesothelioma are diagnosed in the United States each year. Mesothelioma occurs more often in men than in women and risk increases with age, but this disease can appear in either men or women at any age.

Shortness of breath and pain in the chest due to an accumulation of fluid in the pleura are often symptoms of pleural mesothelioma. Symptoms of peritoneal mesothelioma include weight loss and abdominal pain and swelling due to a buildup of fluid in the abdomen. Other symptoms of peritoneal mesothelioma may include bowel obstruction, blood clotting abnormalities, anemia, and fever. If the cancer has spread beyond the mesothelium to other parts of the body, symptoms may include pain, trouble swallowing, or swelling of the neck or face.

The prognosis for mesothelioma is dismal, with poor response to radical surgery, current chemotherapy, radiation therapy, and combination therapy. Microscopic spread of cancer cells into the chest wall and diaphragm are common even when such spread cannot be detected by routine tests.

For many years there have been two principal strategies for chemotherapeutic treatment of cancer: 1) blocking hormone-dependent tumor cell proliferation by interference with the production or peripheral action of sex hormones; and 2) killing cancer cells directly by exposing them to cytotoxic substances, which injure both neoplastic and normal cell populations.

Despite many advances in the field of oncology, the majority of solid tumors remain incurable in the advanced stages. Cytotoxic therapy is used in most cases, however, it often causes significant morbidity to the patient without significant clinical benefit. Less toxic and more specific agents to treat and control advanced malignancies are needed.

An alternate approach for cancer chemotherapy is induction of terminal differentiation of the neoplastic cells (1). In cell culture models differentiation has been reported by exposure of cells to a variety of stimuli, including: cyclic AMP and retinoic acid (2,3), aclarubicin and other anthracyclines (4).

There is abundant evidence that neoplastic transformation does not necessarily destroy the potential of cancer cells to differentiate (1,5, 6). There are many examples of tumor cells which do not respond to the normal regulators of proliferation and appear to be blocked in the expression of their differentiation program, and yet can be induced to differentiate and cease replicating. A variety of agents, including some relatively simple polar compounds (5,7-9), derivatives of vitamin D and retinoic acid (10-12), steroid hormones (13), growth factors (6,14), proteases (15,16), tumor promoters (17, 18), and inhibitors of DNA or RNA synthesis (4,19-24), can induce various transformed cell lines and primary human tumor explants to express more differentiated characteristics.

Early studies identified a series of polar compounds that were effective inducers of differentiation in a number of transformed cell lines (8, 9). Of these, the most effective inducer was the hybrid polar/apolar compound N, N'-hexamethylene bisacetamide (HMBA) (9). The use of this polar/apolar compound to induce murine erythroleukemia cells (MELC) to undergo erythroid differentiation with suppression of oncogenicity has proved a useful model to study inducer-mediated differentiation of transformed cells (5,7-9). HMBA-induced MELC terminal erythroid differentiation is a multi-step process. Upon addition of HMBA to MELC (745A-DS19) in culture, there is a latent period of 10 to 12 hours before commitment to terminal differentiation is detected. Commitment is defined as the capacity of cells to express terminal differentiation despite removal of inducer (25). Upon continued exposure to HMBA there is progressive recruitment of cells to differentiate. The present inventors have reported that MELC cell lines made resistant to relatively low levels of vincristine become markedly more sensitive to the inducing action of HMBA and can be induced to differentiate with little or no latent period (26).

HMBA is capable of inducing phenotypic changes consistent with differentiation in a broad variety of cells lines (5). The characteristics of the drug-induced effect have been most extensively studied in the murine erythroleukemia cell system (MELC) (5,25,27,28). MELC induction of differentiation is both time and concentration dependent. The minimum concentration required to demonstrate an effect in vitro in most strains is 2 to 3 mM; the minimum duration of continuous exposure generally required to induce differentiation in a substantial portion (> 20%) of the population without continuing drug exposure is about 36 hours.

The primary target of action of HMBA is not known. There is evidence that protein kinase C is involved in the pathway of inducer-mediated differentiation (29). The in vitro studies provided a basis for evaluating the potential of HMBA as a cytodifferentiation agent in the treatment of human cancers (30). Several phase I clinical trials with HMBA have been completed (31-36). Clinical trials have shown that this compound can induce a therapeutic response in patients with cancer (35,36). However, these phase I clinical trials also have demonstrated that the potential efficacy of HMBA is limited, in part, by dose-related toxicity which prevents achieving optimal blood levels and by the need for intravenous administration of large quantities of the agent, over prolonged periods.

It has been reported that a number of compounds related to HMBA with polar groups separated by apolar linkages that, on a molar basis, are as active (37) or 100 times more active than HMBA (38). As a class, however, it has been found that the symmetrical dimers such as HMBA and related compounds are not the best cytodifferentiating agents.

It has unexpectedly been found that the best compounds comprise two polar end groups separated by a flexible chain of methylene groups, wherein one or both of the polar end groups is a large hydrophobic group. Preferably, the polar end groups are different and only one is a large hydrophobic group. These compounds are unexpectedly a thousand times more active than HMBA and ten times more active than HMBA related compounds.

Histone deacetylase inhibitors such as suberoylanilide hydroxamic acid (SAHA), belong to this class of agents that have the ability to induce tumor cell growth arrest, differentiation, and/or apoptosis (39). These compounds are targeted towards mechanisms inherent to the ability of a neoplastic cell to become malignant, as they do not appear to have toxicity in doses effective for inhibition of tumor growth in animals (40). There are several lines of evidence that histone acetylation and deacetylation are mechanisms by which transcriptional regulation in a cell is achieved (41). These effects are thought to occur through changes in the structure of chromatin by altering the affinity of histone proteins for coiled DNA in the nucleosome.

There are five types of histones that have been identified (designated H1, H2A, H2B, H3 and H4). Histones H2A, H2B, H3, and H4 are found in the nucleosomes and H1 is a linker located between nucleosomes. Each nucleosome contains two of each histone type within its core, except for H1, which is present singly in the outer portion of the nucleosome structure. It is believed that when the histone proteins are hypoacetylated, there is a greater affinity of the histone to the DNA phosphate backbone. This affinity causes DNA to be tightly bound to the histone and renders the DNA inaccessible to transcriptional regulatory elements and machinery.

The regulation of acetylated states occurs through the balance of activity between two enzyme complexes, histone acetyl transferase (HAT) and histone deacetylase (HDAC). The hypoacetylated state is thought to inhibit transcription of associated DNA. This hypoacetylated state is catalyzed by large multiprotein complexes that include HDAC enzymes. In particular, HDAC have been shown to catalyze the removal of acetyl groups from the chromatin core histones.

The inhibition of HDAC by SAHA is thought occur through direct interaction with the catalytic site of the enzyme as demonstrated by X-ray crystallography studies (42). The result of HDAC inhibition is not believed to have a generalized effect on the genome, but rather, only affects a small subset of the genome (43). Evidence provided by DNA microarrays using malignant cell lines cultured with a HDAC inhibitor shows that there are a finite (1-2%) number of genes whose products are altered. For example, cells treated in culture with HDAC inhibitors show a consistent induction of the cyclin-dependent kinase inhibitor p21 (44). This protein plays an important role in cell cycle arrest. HDAC inhibitors are thought to increase the rate of transcription of p21 by propagating the hyperacetylated state of histones in the region of the p21 gene, thereby making the gene accessible to transcriptional machinery. Genes whose expression is not affected by HDAC inhibitors do not display changes in the acetylation of regional associated histones (45).

It has been shown in several instances that the disruption of HAT or HDAC activity is implicated in the development of a malignant phenotype. For instance, in acute promyelocytic leukemia, the oncoprotein produced by the fusion of PML and RAR alpha appears to suppress specific gene transcription through the recruitment of HDACs (46). In this manner, the neoplastic cell is unable to complete differentiation and leads to excess proliferation of the leukemic cell line.

U.S. Patent Numbers 5,369,108, 5,932,616, 5,700,811, 6,087,367 and 6,511, 990, issued to some of the present inventors, disclose compounds useful for selectively inducing terminal differentiation of neoplastic cells, which compounds have two polar end groups separated by a flexible chain of methylene groups or a by a rigid phenyl group, wherein one or both of the polar end groups is a large hydrophobic group. Some of the compounds have an additional large hydrophobic group at the same end of the molecule as the first hydrophobic group which further increases differentiation activity about 100 fold in an enzymatic assay and about 50 fold in a cell differentiation assay. Methods of synthesizing the compounds used in the methods and pharmaceutical compositions of this invention are fully described in the aforementioned patents.

In addition to their biological activity as antitumor agents, the compounds disclosed in the aforementioned patents have recently been identified as useful for treating or preventing a wide variety of thioredoxin (TRX) -mediated diseases and conditions, such as inflammatory diseases, allergic diseases, autoimmune diseases, diseases associated with oxidative stress of diseases characterized by cellulora hyperproliferation (U.S. Application No. 10/369,094, filed February 15,2003. Further, these compounds have been identified as useful for treating diseases of the central nervous system (CNS) such as neurodegenerative diseases and for treating brain cancer (See, U.S. Application No. 10. 273,401, filed October 16,2002).

The aforementioned patents do not disclose specific oral formulations of the HDAC inhibitors or specific dosages and dosing schedules of the recited compounds that are effective at treating cancer, e.g., mesothelioma. Importantly, the aforementioned patents do not disclose oral formulations that have favourable pharmacokinetic profiles such as high bioavailability which gives rise to high blood levels of the active compounds over an extended period of time.

There is an urgent need to discover suitable dosages and dosing schedules of these compounds, and to develop formulations, preferably oral formulations, which give rise to steady, therapeutically effective blood levels of the active compounds over an extended period of time, and which are effective at treating cancer.

Kelly W. et al., Eur. J. Canc., vol 38, 2003, abstr. 2321 relates to a phase 1 clinical trial of an oral formulation of SAHA.

Heaney M. et al, Proc. Am. Soc. Clin. Oncol. 22, 2003, abstr. 2321 relates to clinical experience with SAHA in heavily pre-treated patients with haematological malignancies.

Kelly W.K. et al., Exp. Op. Invest. Drugs, vol. 11, no. 12, 2002, pages 1695-1713, relates to histone deacetylase inhibitors and to their use in clinical trials.

Cao X.X. et al., Am J. Resp. Cell Mol. Biol., vol. 25, no. 5, 2001, pages 562-568 reports on histone deacetylase inhibitor down regulation of bcl-xl gene expression and apoptotic cell death in mesothelioma.

### SUMMARY OF THE INVENTION

The present invention relates to the use of suberoylanilide hydroxamic acid (SAHA) represented by the structure: 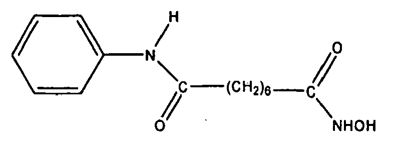 or a pharmaceutically acceptable salt or hydrate thereof, and a pharmaceutically acceptable carrier or diluent, for the preparation of an oral medicament for administration to a subject for the treatment of cancers, e.g., mesothelioma. More specifically, the present invention relates to methods of treating mesothelioma by administration of pharmaceutical compositions comprising HDAC inhibitors, e.g., suberoylanilide hydroxamic acid (SAHA).

The oral formulations of the pharmaceutical compositions have favorable pharmacokinetic profiles such as high bioavailability and surprisingly give rise to high blood levels of the active compounds over an extended period of time. The present invention further provides a safe, daily dosing regimen of these pharmaceutical compositions, which is easy to follow, and which results in a therapeutically effective amount of the HDAC inhibitors *in vivo*.

The present invention provides a method of treating mesothelioma in a subject in need thereof, by administering to the subject a pharmaceutical composition comprising an effective amount of suberoylanilide hydroxamic acid (SAHA) or a pharmaceutically acceptable salt or hydrate thereof, as described herein. SAHA can be administered in a total daily dose of up to 800mg, preferably orally, once, twice, or three times daily, continuously (every day) or intermittently (e.g., 3-5 days a week).

Oral SAHA has been safely administered in phase I clinical studies to patients suffering from mesothelioma or DLBCL.

Furthermore, the present invention provides the use of a pharmaceutical composition comprising an effective amount of an HDAC inhibitor as described herein, or a pharmaceutically acceptable salt or hydrate thereof for the preparation of a medicament for administration to a subject in need thereof for the treatment of mesothelioma. The HDAC inhibitor can be administered in a total daily dose of up to 800 mg, preferably orally, once, twice or three times daily, continuously (i.e., every day) or intermittently (e. g., 3-5 days a week).

The HDAC inhibitors are useful at treating mesothelioma.

HDAC inhibitors suitable for use in the present invention include but are not limited to hydroxamic acid derivatives, Short Chain Fatty Acids (SCFAs), cyclic tetrapeptides, benzamide derivatives, or electrophilic ketone derivatives, as defined herein. Examples of HDAC inhibitors suitable for use in the methods of the present invention are:
A) **Hydroxamic acid derivatives** selected from m-carboxycinnamic acid bishydroxamide (CBHA), Trichostatin A (TSA), Trichostatin C, Salicylhydroxamic Acid, Azelaic Bishydroxamic Acid (ABHA), Azelaic-1-Hydroxamate-9-Anilide (AAHA), 6- (3-Chlorophenylureido) carpoic Hydroxamic Acid (3Cl-UCHA), Oxamflatin, A-161906, Scriptaid, PXD-101, LAQ-824, CHAP, MW2796, and MW2996.

### Specific HDAC inhibitors include, for example:

Suberoylanilide hydroxamic acid (SAHA), which is represented by the following structural formula:

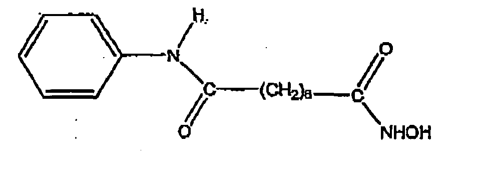

Pyroxamide, which is represented by the following structural formula:

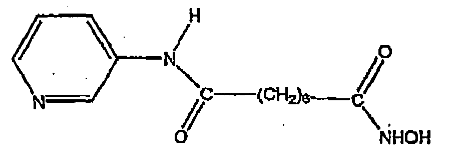

m-Carboxycinnamic acid bishydroxamide (CBHA), which is represented by the structural formula:

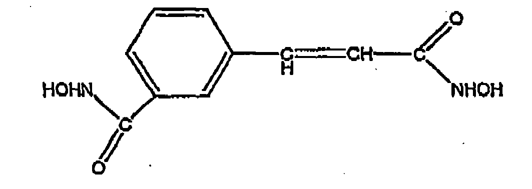

The pharmaceutical compositions comprising the HDAC inhibitor are administered orally, for example within a gelatin capsule. In a further embodiment, the pharmaceutical compositions are further comprised of microcrystalline cellulose, croscarmellose sodium and magnesium stearate.

The HDAC inhibitors can be administered in a total daily dose that may vary from patient to patient, and may be administered at varying dosage schedules. Suitable dosages are total daily dosage of between about 25-4000 mg/m² administered orally once-daily, twice-daily, or three times-daily, continuous (every day) or intermittently (e.g., 3-5 days a week). Furthermore, the compositions may be administered in cycles, with rest periods in between the cycles (e.g., treatment for two to eight weeks with a rest period of up to a week between treatments).

Preferably, the composition is administered once daily at a dose of about 200-600 mg. Preferably, the composition is administered twice daily at a dose of about 200-400 mg. Preferably, the composition is administered twice daily at a dose of about 200-400 mg intermittently, for example three, four, or five days per week. Preferably, the composition is administered three times daily at a dose of about 100-250 mg.

Preferably, the daily dose is 200 mg, which can be administered once-daily, twice-daily, or three-times daily. In one embodiment, the daily dose is 300 mg, which can be administered once-daily or twice-daily. Preferably, the daily dose is 400 mg, which can be administered once-daily or twice-daily.

Also described herein are methods for selectively inducing terminal differentiation, cell growth arrest and/or apoptosis of neoplastic cells, e.g., lymphoma cells in a subject, thereby inhibiting proliferation of such cells in said subject, by administering to the subject a pharmaceutical composition comprising an effective amount of an HDAC inhibitor, e.g., SAHA, or a pharmaceutically acceptable salt or hydrate thereof, and a pharmaceutically acceptable carrier or diluent. An effective amount of an HDAC inhibitor in the present invention can be up to a total daily dose of 800 mg.

Also described herein are methods for inhibiting the activity of a histone deacetylase in a subject, by administering to the subject a pharmaceutical composition comprising an effective amount of an HDAC inhibitor, e.g., SAHA, or a pharmaceutically acceptable salt or hydrate thereof, and a pharmaceutically acceptable carrier or diluent. An effective amount of an HDAC inhibitor can be up to a total daily dose of 800 mg.

Also described herein are *in-vitro* methods for selectively inducing terminal differentiation, cell growth arrest and/or apoptosis of neoplastic cells, e.g., lymphoma cells, thereby inhibiting proliferation of such cells, by contacting the cells with an effective amount of a an HDAC inhibitor, e.g., SAHA, or a pharmaceutically acceptable salt or hydrate thereof.

Also described herein are *in-vitro* methods for inhibiting the activity of a histone deacetylase, by the histone deacetylase with an effective amount of an HDAC inhibitor, e.g., SAHA, or a pharmaceutically acceptable salt or hydrate thereof.

Also described herein is a safe, daily dosing regimen of the formulation of pharmaceutical compositions comprising an HDAC inhibitor that is easy to follow and to adhere to. These pharmaceutical compositions are suitable for oral administration and are useful for treating cancer, e.g., mesothelioma, by selectively inducing terminal differentiation, cell growth arrest, and/or apoptosis of neoplastic cells, and/or-by inhibiting histone deacetylase (HDAC).

### BRIEF DESCRIPTION OF THE DRAWINGS

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.
- **FIG. 1**: is a picture of a Western blot (top panel) showing the quantities of acetylated histone-4 (α-AcH4) in the blood plasma of patients following an oral or intravenous (IV) dose of SAHA. IV SAHA was administered at 200 mg infused over two hours. Oral SAHA was administered in a single capsule at 200 mg. The amount of α-AcH4 is shown at the indicated time points.
Bottom panel: Coomassie blue stain.
- **FIG. 2**: is a picture of a Western blot (top panels) showing the quantities of acetylated histone-4 (α-AcH4) in the blood plasma of patients having a solid tumor, following an oral or intravenous (IV) dose of SAHA. IV and Oral SAHA were administered as in Figure 1. The amount of α-AcH4 is shown at the indicated time points. The experiment is shown in duplicate (Fig 2A and Fig 2B). Bottom panels: Coomassie blue stain.
- **FIG. 3**: is a picture of a Western blot (top panels) showing the quantities of acetylated histone-4 (α -AcH4) (Figure 3A) and acetylated histone-3 (α- AcH3) (Figures 3B-E) in the blood plasma of patients following an oral or intravenous (IV) dose of SAHA, on Day 1 and Day 21. IV and Oral SAHA were administered as in Figure 1. The amount of α-AcH4 or α-AcH3 is shown at the indicated time points. Bottom panels: Coomassie blue stain.
- **FIG. 4**: is a picture of a Western blot (top panels) showing the quantities of acetylated histone-3 (α-AcH3) in the blood plasma of patients having a solid tumor, following an oral or intravenous (IV) dose of SAHA. IV and Oral SAHA were administered as in Figure 1. The amount of α-AcH3 is shown at the indicated time points. Bottom panel: Coomassie blue stain.
- **FIG. 5**: is a picture of a Western blot (top panels) showing the quantities of acetylated histone-3 (α-AcH3) in the blood plasma of patients following an oral or intravenous (IV) dose of SAHA. IV SAHA was administered at 400 mg infused over two hours. Oral SAHA was administered in a single capsule at 400 mg. The amount of α-AcH4 is shown at the indicated time points. The experiment is shown in triplicate (Fig 5A and B). Bottom panels: Coomassie blue stain.
- **FIG. 6**: is a picture of a Western blot (top panel) showing the quantities of acetylated histone-3 (α-AcH3) in the blood plasma of patients having a solid tumor, following an oral or intravenous (IV) dose of SAHA. IV and Oral SAHA were administered as in Figure 5. The amount of α-AcH3 is shown at the indicated time points. Bottom panel: Coomassie blue stain.
- **FIG. 7**: is a picture of a Western blot (top panels) showing the quantities of acetylated histone-3 (α-AcH3) in the blood plasma of patients having a solid tumor following an oral or intravenous (IV) dose of SAHA, on Day 1 and Day 21. IV and Oral SAHA were administered as in Figure 4. The amount of α-AcH4 or α-AcH3 is shown at the indicated time points. The experiment is shown in triplicate (Fig 7A-C). Bottom panels: Coomassie blue stain.
- **FIG. 8**: is a picture of a Western blot (top panels) showing the quantities of acetylated histone-3 (α-AcH3) in the blood plasma of patients following an oral or intravenous (IV) dose of SAHA. IV and Oral SAHA were administered as in Figure 5. The amount of α-AcH3 is shown at the indicated time points. Bottom panels: Coomassie blue stain.
- **FIGS. 9A-C**: are graphs showing the mean plasma concentration of SAHA (ng/ml) at the indicated time points following administration. Fig 9A: Oral dose (200 mg and 400 mg) under fasting on Day 8. Fig 9B: Oral dose (200 mg and 400 mg) with food on Day 9. Fig 9C : IV dose on day 1.
- **FIG. 10**: shows the apparent half-life of a SAHA 200 mg and 400 mg oral dose, on Days 8, 9 and 22.
- **FIG. 11**: shows the AUC (ng/ml/hr) of a SAHA 200 mg and 400 mg oral dose, on Days 8,9 and 22.
- **FIG. 12**: shows the bioavailability of SAHA after a 200 mg and 400 mg oral dose, on Days 8,9 and 22.
- **FIG. 13**: is a CT Scan of a mesothelioma tumor of a patient, before (left) and after (right) six months of treatment with SAHA at a dose of 300 mg twice daily 3 days a week.

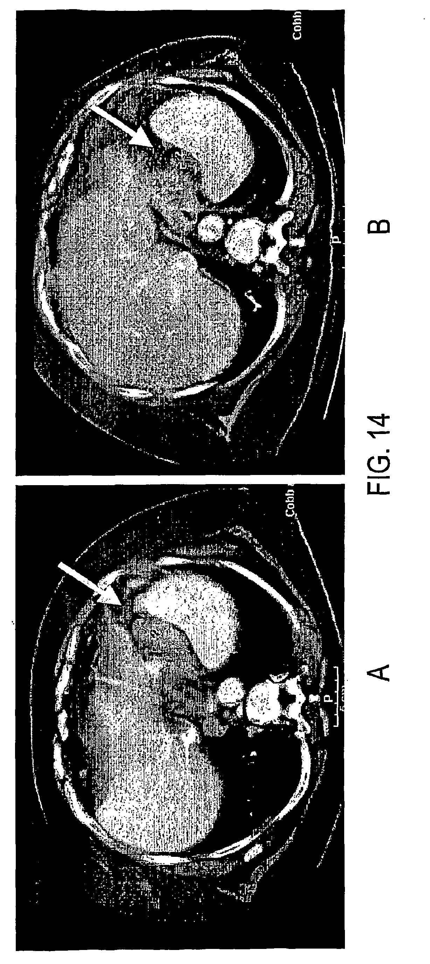

- **FIG. 14**: is a CT scan taken from a patient with Diffuse Large Cell Lymphoma, before treatment (A) and after (B) 2 months of treatment with SAHA at a dose of 400 mg BID for 1 month followed 400 mg QD for one month.

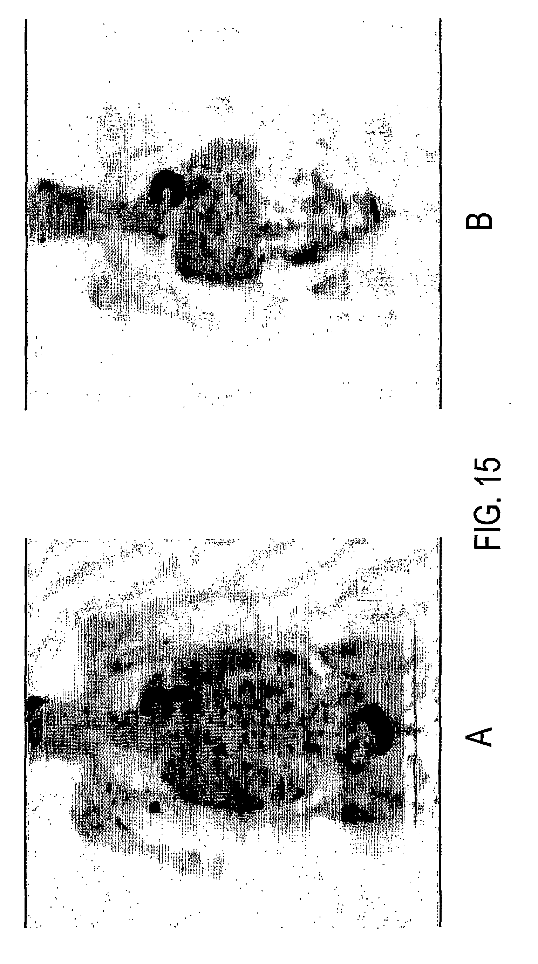

- **FIG. 15**: is a PET scan taken from a patient with Diffuse Large Cell Lymphoma, before treatment (A) and after (B) 2 months of treatment with SAHA at a dose of 400 mg BID for 1 month followed 400 mg QD for one month.

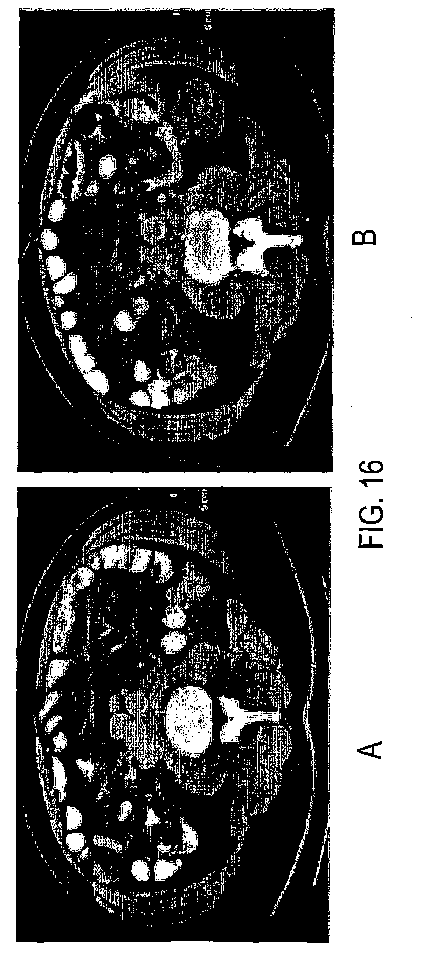

- **FIG. 16**: is a CT scan taken from a patient with Diffuse Large Cell Lymphoma, before treatment (A) and after (B) 1 month of treatment with SAHA at a dose of 600 mg QD.

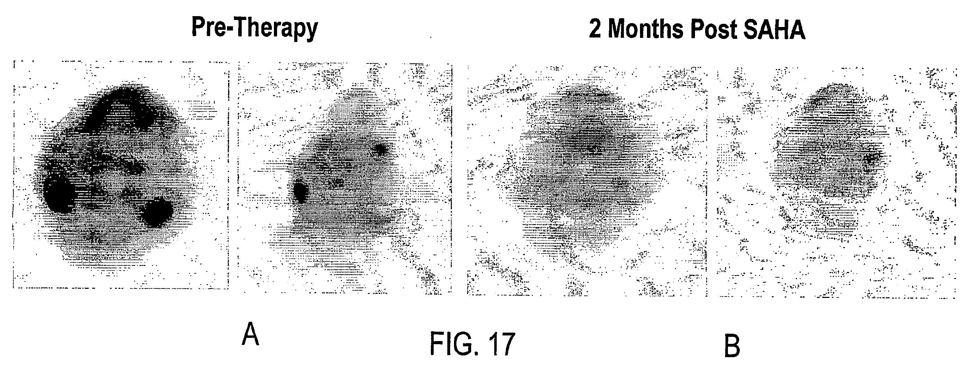

- **FIG. 17**: is a PET scan taken from a patient with Diffuse Large Cell Lymphoma, before treatment (A) and after (B) 2 months of treatment with SAHA at a dose of 200 mg BID.

### DETAILED DESCRIPTION OF THE INVENTION

The present invention relates to pharmaceutical compositions for use in methods of treating cancers, e. g., mesothelioma. More specifically, the present invention relates to the use of pharmaceutical compositions comprising HDAC inhibitors, e.g., suberoylanilide hydroxamic acid (SAHA) for the preparation of a medicament for the treatment of mesothelioma. In specific aspects, the compositions of the invention are used to treat mesothelioma.

The oral formulations of the pharmaceutical compositions of the invention have favorable pharmacokinetic profiles such as high bioavailability and surprisingly give rise to high blood levels of the active compounds over an extended period of time. Thus, the present invention further provides a safe, daily dosing regimen of these pharmaceutical compositions, which is easy to follow, and which results in a therapeutically effective amount of the HDAC inhibitors *in vivo.*

Accordingly, the present invention provides a method of treating mesothelioma in a subject in need thereof, by administering to the subject a pharmaceutical composition comprising an effective amount of an HDAC inhibitor as described herein, or a pharmaceutically acceptable salt or hydrate thereof. The HDAC inhibitor can be administered in a total daily dose of up to 800 mg, preferably orally, once, twice or three times daily, continuously (i. e. , every day) or intermittently (e. g. , 3-5 days a week).

The HDAC inhibitor is suberoylanilide hydroxamic acid (SAHA).

The present invention provides a method of treating mesothelioma in a subject in need thereof, by administering to the subject a pharmaceutical composition comprising an effective amount of suberoylanilide hydroxamic acid (SAHA) or a pharmaceutically acceptable salt or hydrate thereof, as described herein. SAHA, can be administered in a total daily dose of up to 800 mg, preferably orally, once, twice, or three times daily, continuously (every day) or intermittently (e. g. , 3-5 days a week). SAHA is represented by the following structure:

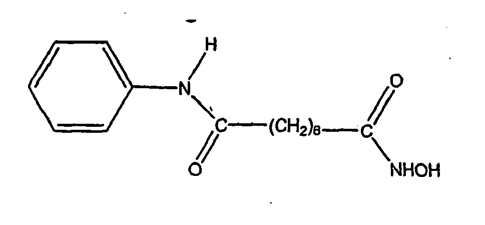

The term "treating" in its various grammatical forms in relation to the present invention refers to preventing (i.e., chemoprevention), curing, reversing, attenuating, alleviating, minimizing, suppressing or halting the deleterious effects of a disease state, disease progression, disease causative agent (e.g., bacteria or viruses) or other abnormal condition. For example, treatment may involve alleviating a symptom (i.e., not necessary all symptoms) of a disease or attenuating the progression of a disease. Because some of the inventive methods involve the physical removal of the etiological agent, the artisan will recognize that they are equally effective in situations where the inventive compound is administered prior to, or simultaneous with, exposure to the etiological agent (prophylactic treatment) and situations where the inventive compounds are administered after (even well after) exposure to the etiological agent

Treatment of cancer, as used herein, refers to partially or totally inhibiting, delaying or preventing the progression of cancer including cancer metastasis; inhibiting, delaying or preventing the recurrence of cancer including cancer metastasis; or preventing the onset or development of cancer (chemoprevention) in a mammal, for example a human.

As used herein, the term "therapeutically effective amount" is intended to encompass any amount that will achieve the desired biological response. In the present invention, the desired biological response is partial or total inhibition, delay, or prevention of the progression of cancer including cancer metastasis; inhibition, delay or prevention of the recurrence of cancer including cancer metastasis; or the prevention of the onset or development of cancer (chemoprevention) in a mammal, for example a human.

The method of the present invention is intended for the treatment or chemoprevention of human patients with cancer. However, it is also likely that the method would be effective in the treatment of cancer in other mammals.

### Histone Deacetylases and Histone Deacetylase Inhibitors

Histone deacetylases (HDACs), as that term is used herein, are enzymes that catalyze the removal of acetyl groups from lysine residues in the amino terminal tails of the nucleosomal core histones. As such, HDACs together with histone acetyl transferases (HATs) regulate the acetylation status of histones. Histone acetylation affects gene expression and inhibitors of HDACs, such as the hydroxamic acid-based hybrid polar compound suberoylanilide hydroxamic acid (SAHA) induce growth arrest, differentiation and/or apoptosis of transformed cells *in vitro* and inhibit tumor growth *in vivo*. HDACs can be divided into three classes based on structural homology. Class I HDACs (HDACs 1, 2, 3 and 8) bear similarity to the yeast RPD3 protein, are located in the nucleus and are found in complexes associated with transcriptional co-repressors. Class II HDACs (HDACs 4, 5, 6, 7 and 9) are similar to the yeast HDA1 protein, and have both nuclear and cytoplasmic subcellular localization. Both Class I and II HDACs are inhibited by hydroxamic acid-based HDAC inhibitors, such as SAHA. Class III HDACs form a structurally distant class of NAD dependent enzymes that are related to the yeast SIR2 proteins and are not inhibited by hydroxamic acid-based HDAC inhibitors.

Histone deacetylase inhibitors or HDAC inhibitors, as that term is used herein are compounds that are capable of inhibiting the deacetylation of histones *in vivo*, *in vitro* or both. As such, HDAC inhibitors inhibit the activity of at least one histone deacetylase. As a result of inhibiting the deacetylation of at least one histone, an increase in acetylated histone occurs and accumulation of acetylated histone is a suitable biological marker for assessing the activity of HDAC inhibitors. Therefore, procedures that can assay for the accumulation of acetylated histones can be used to determine the HDAC inhibitory activity of compounds of interest. It is understood that compounds that can inhibit histone deacetylase activity can also bind to other substrates and as such can inhibit other biologically active molecules such as enzymes. It is also to be understood that the compounds of the present invention are capable of inhibiting any of the histone deacetylases set forth above, or any other histone deacetylases.

For example, in patients receiving HDAC inhibitors, the accumulation of acetylated histones in peripheral mononuclear cells as well as in tissue treated with HDAC inhibitors can be determined against a suitable control.

HDAC inhibitory activity of a particular compound can be determined *in vitro* using, for example, an enzymatic assays which shows inhibition of at least one histone deacetylase. Further, determination of the accumulation of acetylated histones in cells treated with a particular composition can be determinative of the HDAC inhibitory activity of a compound.

Assays for the accumulation of acetylated histones are well known in the literature. See, for example, Marks, P.A. et al., J. Natl. Cancer Inst., 92:1210-1215, 2000, Butler, L.M. et al., Cancer Res. 60:5165-5170 (2000), Richon, V. M. et al., Proc. Natl. Acad. Sci., USA, 95:3003-3007, 1998, and Yoshida, M. et al., J. Biol. Chem., 265:17174-17179, 1990.

For example, an enzymatic assay to determine the activity of an HDAC inhibitor compound can be conducted as follows. Briefly, the effect of an HDAC inhibitor compound on affinity purified human epitope-tagged (Flag) HDAC1 can be assayed by incubating the enzyme preparation in the absence of substrate on ice for about 20 minutes with the indicated amount of inhibitor compound. Substrate ([³H]acetyl-labeled murine erythroleukemia cell-derived histone) can be added and the sample can be incubated for 20 minutes at 37°C in a total volume of 30 µL. The reaction can then be stopped and released acetate can be extracted and the amount of radioactivity release determined by scintillation counting. An alternative assay useful for determining the activity of an HDAC inhibitor compound is the "HDAC Fluorescent Activity Assay; Drug Discovery Kit-AK-500" available from BIOMOL® Research Laboratories, Inc., Plymouth Meeting, PA.

*In vivo* studies can be conducted as follows. Animals, for example, mice, can be injected intraperitoneally with an HDAC inhibitor compound. Selected tissues, for example, brain, spleen, liver etc, can be isolated at predetermined times, post administration. Histones can be isolated from tissues essentially as described by Yoshida et al., J. Biol. Chem. 265:17174-17179, 1990. Equal amounts of histones (about 1 µg) can be electrophoresed on 15% SDS-polyacrylamide gels and can be transferred to Hybond-P filters (available from Amersham). Filters can be blocked with 3% milk and can be probed with a rabbit purified polyclonal anti-acetylated histone H4 antibody (α-Ac-H4) and anti-acetylated histone H3 antibody (α-Ac-H3) (Upstate Biotechnology, Inc.). Levels of acetylated histone can be visualized using a horseradish peroxidase-conjugated goat anti-rabbit antibody (1:5000) and the SuperSignal chemiluminescent substrate (Pierce). As a loading control for the histone protein, parallel gels can be run and stained with Coomassie Blue (CB).

In addition, hydroxamic acid-based HDAC inhibitors have been shown to up regulate the expression of the p21^{WAFI} gene. The p21^{WAFI} protein is induced within 2 hours of culture with HDAC inhibitors in a variety of transformed cells using standard methods. The induction of the p21^{WAFI} gene is associated with accumulation of acetylated histones in the chromatin region of this gene. Induction of p21^{WAFI} can therefore be recognized as involved in the G1 cell cycle arrest caused by HDAC inhibitors in transformed cells.

Typically, HDAC inhibitors fall into five general classes: 1) hydroxamic acid derivatives; 2) Short-Chain Fatty Acids (SCFAs); 3) cyclic tetrapeptides ; 4) benzamides ; and 5) electrophilic ketones.
A. **Hydroxamic Acid Derivatives** such as suberoylanilide hydroxamic acid (SAHA) (Richon et al., Proc. Natl. Acad. Sci. USA 95,3003-3007 (1998)) ; m- carboxycinnamic acid bishydroxamide (CBHA) (Richon et al., supra); pyroxamide ; trichostatin analogues such as trichostatin A (TSA) and trichostatin C (Koghe et al. 1998. Biochem. Pharmacol. 56: 1359-1364); salicylhydroxamic acid (Andrews et al., International J. Parasitology 30, 761-768 (2000) ) ; suberoyl bishydroxamic acid (SAHA) (U. S. Patent No. 5,608, 108) ; azelaic bishydroxamic acid (ABHA) (Andrews et al., supra); azelaic-1- hydroxamate-9-anilide (AAHA) (Qiu et al., Mol. Biol. Cell 11,2069-2083 (2000) ) ; 6- (3- chlorophenylureido) carpoic hydroxamic acid (3C1-UCHA) ; oxamflatin [ (2E)-5- [3- [(phenylsufonyl) aminol phenyl]-pent-2-en-4-ynohydroxamic acid] (Kim et al. Oncogene, 18: 2461 2470 (1999)); A-161906, Scriptaid (Su et al. 2000 Cancer Research, 60: 3137- 3142); PXD-101 (Prolifix) ; LAQ-824; CHAP; MW2796 (Andrews et al., supra); MW2996 (Andrews et al., supra) ; or any of the hydroxamic acids disclosed in U. S. Patent Numbers 5,369,108, 5,932,616, 5,700,811, 6,087,367 and 6,511,990.

SAHA has been shown to bind directly in the catalytic pocket of the histone deacetylase enzyme. SAHA induces cell cycle arrest, differentiation, and/or apoptosis of transformed cells in culture and inhibits tumor growth in rodents. SAHA is effective at inducing these effects in both solid tumors and hematological cancers. It has been shown that SAHA is effective at inhibiting tumor growth in animals with no toxicity to the animal. The SAHA-induced inhibition of tumor growth is associated with an accumulation of acetylated histones in the tumor. SAHA is effective at inhibiting the development and continued growth of carciaogen-induced (N-methylnitrosourea) mammary tumors in rats. SAHA was administered to the rats in their diet over the 130 days of the study. Thus, SAHA is a nontoxic, orally active antitumor agent whose mechanism of action involves the inhibition of histone deacetylase activity.

Preferred HDAC inhibitors are those disclosed in U.S. Patent Numbers 5,369,108, 5,932,616, 5,700,811, 6,087,367 and 6,511, 990, issued to some of the present inventors disclose compounds.

The HDAC inhibitor used in the present invention is represented by the structure of formula 3, or a pharmaceutically acceptable salt or hydrate thereof, and a pharmaceutically acceptable carrier or excipient. 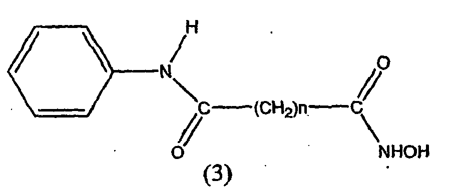 wherein n is an integer from 5 to about 8.

In a preferred embodiment of formula 3, n is 6. The HDAC inhibitor is SAHA (4), or a pharmaceutically acceptable salt or hydrate thereof, and a pharmaceutically acceptable carrier or excipient. SAHA can be represented by the following structural formula:

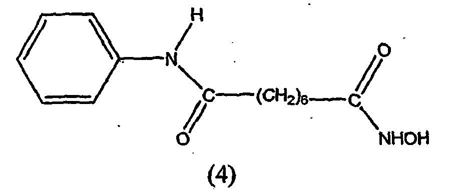

Also disclosed herein are pharmaceutical compositions comprising pharmaceutically acceptable salts of the HDAC inhibitors with organic and inorganic acids, for example, acid addition salts which may, for example, be hydrochloric acid, sulphuric acid, methanesulphonic acid, fumaric acid, maleic acid, succinic acid, acetic acid, benzoic : acid, oxalic acid, citric acid, tartaric acid, carbonic acid, phosphoric acid and the like. Pharmaceutically acceptable salts can also be prepared from by treatment with inorganic bases, for example, sodium, potassium, ammonium, calcium, or ferric hydroxides, and such organic bases as isopropylamine, trimethylamine, 2-ethylamino ethanol, histidine, procaine, and the like.

Also disclosed herein are pharmaceutical compositions comprising hydrates of the HDAC inhibitors. The term"hydrate"includes but is not limited to hemihydrate, monohydrate, dihydrate, trihydrate, and the like.

This invention also encompasses pharmaceutical compositions comprising any solid or liquid physical form of SAHA. The HDAC inhibitors can be in a crystalline form, in amorphous form, and have any particle size. The HDAC inhibitor particles may be micronized, or may be agglomerated, particulate granules, powders, oils, oily suspensions, or any other form of solid or liquid physical form.

### Therapeutic Uses of HDAC Inhibitors

### 1. Treatment of Cancer

As demonstrated herein, the HDAC inhibitors are useful for the treatment of cancer comprising administering to said subject a therapeutically effective amount of a histone deacetylase inhibitor described herein.

The term "cancer" refers to cancer caused by the proliferation of neoplastic cells, such as mesotheliomas, for example, mesotheliomas such as pleural mesothelioma, peritoneal mesothelioma, and benign fibrous mesothelioma.

### 2. Treatment of Mesothelioma

As demonstrated herein, the HDAC inhibitors are useful for the treatment of mesothelioma.

There are various types of mesothelioma. Pleural and peritoneal (malignant) mesotheliomas involve tumors of mesothelial tissue associated with asbestos exposure. Histologically, these tumors are composed of epitheloid, sarcomatoid, or fibrous, or mixed cell types (also called biphasic type). Benign fibrous mesothelioma is a rare solid tumor of the pleura that produces chest pain, dyspnea, fever, and hypertrophic osteoarthropathy.

One staging system used for mesothelioma is the Butchart system. This system is based mainly on the extent of the primary malignant tumor mass, and divides mesotheliomas into stages I through IV.

| |
|---|
| Stage I : Mesothelioma is present on one side of the chest only and isn't growing into the chest wall. |
| Stage II : Mesothelioma invades the chest wall or involves the esophagus (food passage connecting the throat to the stomach), heart, or has grown into the pleura on the other side of the chest. The lymph nodes in the chest may also be involved. |
| Stage III: Mesothelioma has grown through the diaphragm into the peritoneum (lining of the abdominal cavity) or has spread to lymph nodes beyond those in the chest. |
| Stage IV: Mesothelioma has spread through the bloodstream to other organs (metastases). |

### Grading

As contemplated herein, the HDAC inhibitors of the present invention are useful at treating all of the stages of mesothelioma i.e., stages I, III, III and IV stated above.

### Combination therapy

The uses of the present invention may also comprise initially administering to the subject an antitumor agent so as to render the neoplastic cells in the subject resistant to an antitumor agent and subsequently administering an effective amount of any of the compositions of the present invention, effective to selectively induce terminal differentiation, cell growth arrest and/or apoptosis of such cells, or to treat cancer or provide chemoprevention.

The antitumor agent may be one of numerous chemotherapy agents such as an alkylating agent, an antimetabolite, a hormonal agent, an antibiotic, colchicine, a vinca alkaloid, L-asparaginase, procarbazine, hydroxyurea, mitotane, nitrosoureas, or an imidazole carboxamide. Suitable agents are those agents that promote depolarization of tubulin. Preferably the antitumor agent is colchicine or a vinca alkaloid ; especially preferred are vinblastine and vincristine. In embodiments where the antitumor agent is vincristine, the cells preferably are treated so that they are resistant to vincristine at a concentration of about 5 mg/ml. The treating of the cells to render them resistant to an antitumor agent may be effected by contacting the cells with the agent for a period of at least 3 to 5 days. The contacting of the resulting cells with any of the compounds above is performed as described previously. In addition to the above chemotherapy agents, the compounds may also be administered together with radiation therapy.

### Dosages and Dosage Schedules

The dosage regimen utilizing the HDAC inhibitors can be selected in accordance with a variety of factors including type, species, age, weight, sex and the type of cancer being treated; the severity (i. e. , stage) of the cancer to be treated; the route of administration; the renal and hepatic function of the patient; and the particular compound or salt thereof employed. An ordinarily skilled physician or veterinarian can readily determine and prescribe the effective amount of the drug required to treat, for example, to prevent, inhibit (fully or partially) or arrest the progress of the disease.

Suitable dosages are total daily dosage of between about 25-4000 mg/m² administered orally once-daily, twice-daily or three times-daily, continuous (every day) or intermittently (e.g., 3-5 days a week). For example, SAHA can be administered in a total daily dose of up to 800 mg. The HDAC inhibitor can be administered once daily (QD), or divided into multiple daily doses, such as twice daily (BID), and three times daily (TID). The HDAC inhibitor can be administered at a total daily dosage of up to 800 mg, e. g. , 150 mg, 200 mg, 300 mg, 400 mg, 600 mg or 800 mg, which can be administered in one daily dose or can be divided into multiple daily doses as described above. Preferably, the administration is oral.

The composition can be administered once daily at a dose of about 200-600 mg; twice daily at a dose of about 200-400 mg; twice daily at a dose of about 200-400 mg intermittently, for example three, four, or five days per week; and three times daily at a dose of about 100-250 mg.

The daily dose is 200 mg, which can be administered once-daily, twice-daily, or three-times daily; the daily dose can be 300 mg, which can be administered once-daily, twice-daily, or three-times daily; the daily dose can be 400 mg, which can be administered once-daily or twice-daily; the daily dose can be 150 mg, which can be, administered twice-daily or three-times daily.

In addition, the administration can be continuous, i.e., every day, or intermittently. The terms"intermittent"or"intermittently"as used herein means stopping and starting at either regular or irregular intervals. For example, intermittent administration of an HDAC inhibitor may be administration one to six days per week or it may mean administration in cycles (e. g. , daily administration for two to eight consecutive weeks, then a rest period with no administration for up to one week) or it may mean administration on alternate days.

SAHA or any of the HDAC inhibitors can be administered to the patient at a total daily dosage between 25-4000 mg/m².

A treatment protocol comprises continuous administration (i.e., every day), once, twice, or three times daily at a total daily dose in the range of about 200 mg to about 600 mg.

Another treatment protocol comprises intermittent administration of between three to five days a week, once, twice or three times daily at a total daily dose in the range of about 200 mg to about 600. mg.

The HDAC inhibitor can be administered continuously once daily at a dose of 400 mg or twice daily at a dose of 200 mg.

The HDAC inhibitor can be administered intermittently three days a week, once daily at a dose of 400 mg or twice daily at a dose of 200 mg; four days a week, once daily at a dose of 400 mg or twice daily at a dose of 200 mg; five days a week, once daily at a dose of 400 mg or twice daily at a dose of 200 mg; or administered continuously once daily at a dose of 600 mg, twice daily at a dose of 300 mg, or three times daily at a dose of 200 mg.

The HDAC inhibitor can be administered intermittently three days a week, once daily at a dose of 600 mg, twice daily at a dose of 300 mg, or three times daily at a dose of 200 mg; four days a week, once daily at a dose of 600 mg, twice daily at a dose of 300 mg, or three times daily at a dose of 200 mg; or five days a week, once daily at a dose of 600 mg, twice daily at a dose of 300 mg, or three times daily at a dose of 200 mg.

In addition, the HDAC inhibitor may be administered according to any of the schedules described above, consecutively for a few weeks, followed by a rest period. For example, the HDAC inhibitor may be administered according to any one of the schedules described above from two to eight weeks, followed by a rest period of one week, or twice daily at a dose of 300 mg for three to five days a week. The HDAC inhibitor can be administered three times daily for two consecutive weeks, followed by one week of rest.

The present invention provides a safe, daily dosing regimen of these formulations, which is easy to follow and to adhere to. The formulations of the the compositions of the present invention are therefore useful for selectively inducing terminal differentiation, cell growth arrest, and/or apoptosis of neoplastic cells and therefore aid in treatment of tumors.

### Pharmaceutical compositions

The compounds of the invention, and derivatives, fragments, analogs, homologs pharmaceutically acceptable salts or hydrate thereof, can be incorporated into pharmaceutical compositions suitable for oral administration, together with a pharmaceutically acceptable carrier or excipient. Such compositions typically comprise a therapeutically effective amount of any of the compounds above, and a pharmaceutically acceptable carrier. Preferably, the effective amount is an amount effective to selectively induce terminal differentiation of suitable neoplastic cells and less than an amount which causes toxicity in a patient.

The compositions of the present invention may be formulated in any unit dosage form (liquid or solid) suited for oral administration, for example, in the form of a pellet, a tablet, a coated tablet, a capsule, a gelatin capsule, a solution, a suspension, or a dispersion. In a currently preferred embodiment, the composition is in the form of a gelatin capsule.

Any inert excipient that is commonly used as a carrier or diluent may be used in the formulations of the present invention, such as for example, a gum, a starch, a sugar, a cellulosic material, an acrylate, or mixtures thereof. A preferred diluent is microcrystalline cellulose. The compositions may further comprise a disintegrating agent (e. g., croscarmellose sodium) and a lubricant (e. g. , magnesium stearate), and in addition may comprise one or more additives selected from a binder, a buffer, a protease inhibitor, a surfactant, a solubilizing agent, a plasticizer, an emulsifier, a stabilizing agent, a viscosity increasing agent, a sweetener, a film forming agent, or any combination thereof. Furthermore, the compositions of the present invention may be in the form of controlled release or immediate release formulations.

One embodiment is a pharmaceutical composition for oral administration comprising SAHA or a pharmaceutically acceptable salt or hydrate thereof, microcrystalline cellulose, croscarmellose sodium and magnesium stearate. Preferably the composition comprises 50-70% by weight of a HDAC inhibitor or a pharmaceutically acceptable salt or hydrate thereof, 20-40% by weight microcrystalline cellulose, 5-1.5% by weight croscarmellose sodium and 0.1-5% by weight magnesium stearate. The compositions can comprise about 50-200 mg of a HDAC inhibitor.

The pharmaceutical compositions are administered orally, and are thus formulated in a form suitable for oral administration, i. e. , as a solid or a liquid preparation. Suitable solid oral formulations include tablets, capsules, pills, granules, pellets, and the like. Suitable liquid oral formulations include solutions, suspensions, dispersions, emulsions, oils, and the like. In one embodiment of the present invention, the composition is formulated in a capsule. In accordance with this, the compositions may comprise in addition to the HDAC inhibitor active compound and the inert carrier or diluent, a hard gelatin capsule.

As used herein, "pharmaceutically acceptable carrier" is intended to include any and all solvents, dispersion media, coatings, antibacterial and antifungal agents, isotonic and absorption delaying agents, and the like, compatible with pharmaceutical administration, such as sterile pyrogen-free water. Suitable carriers are described in the most recent edition of Remington's Pharmaceutical Sciences, a standard reference text in the field. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

Solid carriers/diluents include, but are not limited to, a gum, a starch (e. g., corn starch, pregelatinized starch), a sugar (e. g. , lactose, mannitol, sucrose, dextrose), a cellulosic material (e. g., microcrystalline cellulose), an acrylate (e. g. , polymethylacrylate), calcium carbonate, magnesium oxide, talc, or mixtures thereof.

For liquid formulations, pharmaceutically acceptable carriers may be aqueous or non-aqueous solutions, suspensions, emulsions, or oils. Examples of non-aqueous solvents are propylene glycol, polyethylene glycol, and injectable organic esters such as ethyl oleate. Aqueous carriers include water, alcoholic/aqueous solutions, emulsions, or suspensions, including saline and buffered media. Examples of oils are those of petroleum, animal, vegetable, or synthetic origin, for example, peanut oil, soybean oil, mineral oil, olive oil, sunflower oil, and fish-liver oil. Solutions or suspensions can also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide.

In addition, the compositions may further comprise binders (e.g., acacia, cornstarch, gelatin, carbomer, ethyl cellulose, guar gum, hydroxypropyl cellulose, hydroxypropyl methyl cellulose, povidone), disintegrating agents (e.g., cornstarch, potato starch, alginic acid, silicon dioxide, croscarmellose sodium, crospovidone, guar gum, sodium starch glycolate, Primogel), buffers (e.g., tris-HCI, acetate, phosphate) of various pH and ionic strength, additives such as albumin or gelatin to prevent absorption to surfaces, detergents (e.g.; Tween 20, Tween 80; Pluronic F68, bile acid salts), protease inhibitors, surfactants (e.g., sodium lauryl sulfate), permeation enhancers, solubilizing agents (e.g., glycerol, polyethylene glycerol), a glidant (e.g., colloidal silicon dioxide), anti-oxidants (e.g., ascorbic acid, sodium metabisulfite, butylated hydroxyanisole), stabilizers (e.g., hydroxypropyl cellulose, hyroxypropylmethyl cellulose), viscosity increasing agents (e.g., carbomer, colloidal silicon dioxide, ethyl cellulose, guar gum), sweeteners (e.g., sucrose, aspartame, citric acid), flavoring agents (e.g., peppermint, methyl salicylate, or orange flavoring), preservatives (e.g., Thimerosal, benzyl alcohol, parabens), lubricants (e.g., stearic acid, magnesium stearate, polyethylene glycol, sodium lauryl sulfate), flow-aids (e.g., colloidal silicon dioxide), plasticizers (e.g., diethyl phthalate, triethyl citrate), emulsifiers (e.g., carbomer, hydroxypropyl cellulose, sodium lauryl sulfate), polymer coatings (e.g., poloxamers or poloxamines), coating and film forming agents (e.g., ethyl cellulose, acrylates, polymethacrylates) and/or adjuvants.

Preferably, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The pharmaceutical compositions can be included in a container, pack, or dispenser together with instructions for administration.

The daily administration can be repeated continuously for a period of several days to several years. Oral treatment may continue for between one week and the life of the patient. Preferably the administration takes place for five consecutive days after which time the patient can be evaluated to determine if further administration is required. The administration can be continuous or intermittent, i.e., treatment for a number of consecutive days followed by a rest period.

The compositions of the present invention may be administered intravenously on the first day of treatment, with oral administration on the second day and all consecutive days thereafter.

The compositions of the present invention may be administered for the purpose of preventing disease progression or stabilizing tumor growth.

The preparation of pharmaceutical compositions that contain an active component is well understood in the art, for example, by mixing, granulating, or tablet-forming processes. The active therapeutic ingredient is often mixed with excipients that are pharmaceutically acceptable and compatible with the active ingredient. For oral administration, the active agents are mixed with additives customary for this purpose, such as vehicles, stabilizers, or inert diluents, and converted by customary methods into suitable forms for administration, such as tablets, coated tablets, hard or soft gelatin capsules, aqueous, alcoholic or oily Solutions and the like as detailed above.

The amount of the compound administered to the patient is less than an amount that would cause toxicity in the patient. The amount of the compound that is administered to the patient can be less than the amount that causes a concentration of the compound in the patient's plasma to equal or exceed the toxic level of the compound. Preferably, the concentration of the compound in the patient's plasma is maintained at about 10 nM. The concentration of the compound in the patient's plasma can be maintained at about 25 nM. The concentration of the compound in the patient's plasma can be maintained at about 50 nM. The concentration of the compound in the patient's plasma can be maintained at about 100 nM. The concentration of the compound in the patient's plasma can be maintained at about 500 nM. The concentration of the compound in the patient's plasma can be maintained at about 1000 nM. The concentration of the compound in the patient's plasma can be maintained at about 2500 nM. The concentration of the compound in the patient's plasma can be maintained at about 5000 nM.

It has been found with HMBA that administration of the compound in an amount from about 5 gm/m²/day to about 30 gm/m²/day, particularly about 20 gm/m²/day, is effective without producing toxicity in the patient. The optimal amount of the compound that should be administered to the patient in the practice of the present invention will depend on the particular compound used and the type of cancer being treated.

In a currently preferred embodiment of the present invention, the pharmaceutical composition comprises suberoylanilide hydroxamic acid (SAHA); microcrystalline cellulose as a carrier or diluent; croscarmellose sodium as a disintegrant; and magnesium stearate as a lubricant. Another currently preferred embodiment of the invention is a solid formulation of SAHA with microcrystalline cellulose, NF (Avicel Ph 101), sodium croscarmellose, NF (AC-Di-Sol) and magnesium stearate, NF, contained in a gelatin capsule.

The percentage of the active ingredient and various excipients in the formulations may vary. For example, the composition may comprise between 20 and 90%, preferably between 50-70% by weight of the histone deacetylase (HDAC). Furthermore, the composition may comprise between 10 and 70%, preferably between 20-40% by weight microcrystalline cellulose as a carrier or diluent. Furthermore, the composition may comprise between 1 and 30%, preferably 5-15% by weight croscarmellose sodium as a disintegrant. Furthermore, the composition may comprise between 0.1-5% by weight magnesium stearate as a lubricant. In another preferred embodiment, the composition comprises about 50-200 mg of the HDAC inhibitor (e. g. , 50 mg, 100 mg and 200 mg for the HDAC inhibitor, i.e. SAHA). In a particularly preferred embodiment, the composition is in the form of a gelatin capsule.

A currently preferred embodiment is 200 mg of solid SAHA with 89.5 mg of microcrystalline cellulose, 9 mg of sodium croscarmellose and 1.5 mg of magnesium stearate contained in a gelatin capsule.

### In Vitro Uses:

Also described herein are *in-vitro* methods for selectively inducing terminal differentiation, cell growth arrest and/or apoptosis of neoplastic cells, e.g., lymphoma cells, thereby inhibiting proliferation of such cells, by contacting the cells in vitro with an effective amount of a an HDAC inhibitor, e.g., SAHA, or a pharmaceutically acceptable salt or hydrate thereof.

Also described herein are *in*-*vitro* methods for inhibiting the activity of a histone deacetylase, by the histone deacetylase with an effective amount of an HDAC inhibitor, e.g., SAHA, or a pharmaceutically acceptable salt or hydrate thereof.

Thus, the present invention also provides pharmaceutical compositions for use in methods for selectively inducing terminal differentiation, cell growth arrest and/or apoptosis of neoplastic cells, e.g., lymphoma cells in a subject, thereby inhibiting proliferation of such cells in said subject, by administering to the subject said pharmaceutical composition comprising an effective amount of an HDAC inhibitor, e.g., SAHA, or a pharmaceutically acceptable salt or hydrate thereof, and a pharmaceutically acceptable carrier or diluent. An effective amount of an HDAC inhibitor can be up to a total daily dose of 800 mg.

The present invention also provides the use of SAHA in preparation of an oral medicament for inhibiting the activity of a histone deacetylase in a subject, by administering to the subject a pharmaceutical composition comprising an effective amount of SAHA, or a pharmaceutically acceptable salt or hydrate thereof, and a pharmaceutically acceptable carrier or diluent. An effective amount of an HDAC inhibitor in the present invention can be up to a total daily dose of 800 mg.

### EXAMPLES

The invention is illustrated in the Examples that follow. This section is set forth to aid in an understanding of the invention but is not intended to, and should not be construed to limit in any way the invention as set forth in the claims which follow thereafter.

### EXAMPLE 1

### Synthesis of SAHA

SAHA can be synthesized according to the method outlined below, or according to the method set forth in US Patent 5,369, 108, or according to any other method.

### Synthesis of SAHA

### Step 1 - Synthesis of Suberanilic acid

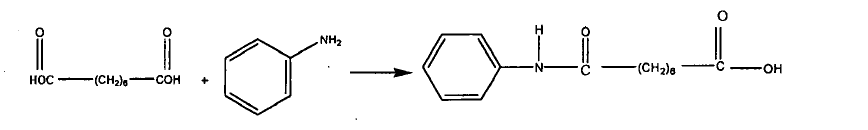

In a 22 L flask was placed 3,500 g (20.09 moles) of suberic acid, and the acid melted with heat. The temperature was raised to 175°C, and then 2,040 g (21.92 moles) of aniline was added. The temperature was raised to 190°C and held at that temperature for 20 minutes. The melt was poured into a Nalgene tank that contained 4,017 g of potassium hydroxide dissolved in 50 L of water. The mixture was stirred for 20 minutes following the addition of the melt. The reaction was repeated at the same scale, and the second melt was poured into the same solution of potassium hydroxide. After the mixture was thoroughly stirred, the stirrer was turned off, and the mixture was allowed to settle. The mixture was then filtered through a pad of Celite (4,200 g) (the product was filtered to remove the neutral by-product (from attack by aniline on both ends of suberic acid). The filtrate contained the salt of the product, and also the salt of unreacted suberic acid. The mixture was allowed to settle because the filtration was very slow, taking several days). The filtrate was acidified using 5 L of concentrated hydrochloric acid; the mixture was stirred for one hour, and then allowed to settle overnight. The product was collected by filtration, and washed on the funnel with deionized water (4 x 5 L). The wet filter cake was placed in a 72 L flack with 44 L of deionized water, the mixture heated to 50°C, and the solid isolated by a hot filtration (the desired product was contaminated with suberic acid which is has a much greater solubility in hot water. Several hot triturations were done to remove suberic acid The product was checked by NMR [D₆DMSO] to monitor the removal of suberic acid) The hot trituration was repeated with 44 L of water at 50°C. The product was again isolated by filtration, and rinsed with 4 L of hot water. It was dried over the weekend in a vacuum oven at 65°C using a Nash pump as the vacuum source (the Nash pump is a liquid ring pump (water) and pulls a vacuum of about 29 inch of mercury. An intermittent argon purge was used to help carry off water); 4,182.8 g of suberanilic acid was obtained.

The product still contained a small amount, of suberic acid; therefore the hot trituration was done portionwise at 65°C, using about 300 g of product at a time. Each portion was filtered, and rinsed thoroughly with additional hot water (a total of about 6 L) This was repeated to purity the entire batch. This completely removed suberic acid from the product. The solid product was combined in a flask and stirred with 6 L of methanol/water (1:2), and then isolated by filtration and air dried on the filter over the week end. It was placed in trays and dried in a vacuum oven at 65°C for 45 hours using the Nash pump and an argon bleed. The final product has a weight of 3,278.4 g (32.7% yield).

### Step 2 -Synthesis of Methyl Suberanilate

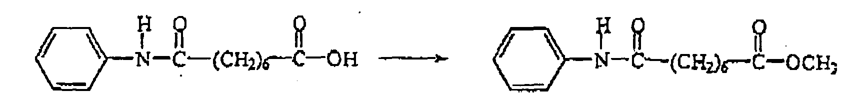

To a 50 L flask fitted with a mechanical stirrer, and condenser was placed 3,229 g of suberanilic acid from the previous step, 20 L of methanol, and 398.7 g of Dowex 50WX2-400 resin. The mixture was heated to reflux and held at reflux for 18 hours. The mixture was filtered to remove the resin beads, and the filtrate was taken to a residue on a rotary evaporator.

The residue from the rotary evaporator was transferred into a 50 L flask fitted with a condenser and mechanical stirrer. To the flask was added 6 L of methanol, and the mixture heated to give a solution. Then 2 L of deionized water was added, and the heat turned off. The stirred mixture was allowed to cool, and then the flask was placed in an ice bath, and the mixture cooled. The solid product was, isolated by filtration, and the filter cake was rinsed with 4 L of cold methanol/water (1:1). The product was dried at 45°C in a vacuum oven using a Nash pump for a total of 64 hours to give 2,850.2 g (84% yield) of methyl suberanilate, CSL Lot # 98-794-92-3 1.

### Step 3 - Synthesis of Crude SAHA

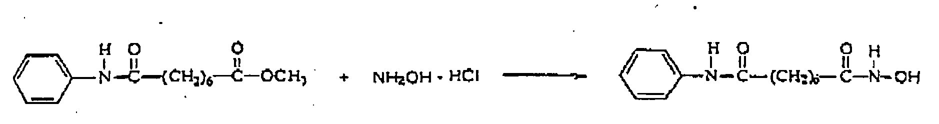

To a 50 L flask with a mechanical stirrer, thermocouple, and inlet for inert atmosphere was added 1,451.9 g of hydroxylamine, hydrochloride, 19 L of anhydrous methanol, and a 3.93 L of a 30% sodium methoxide solution in methanol. The flask was then charged with 2,748.0 g of methyl suberanilate, followed by 1.9 L of a 30% sodium methoxide solution in methanol. The mixture was allowed to stir for 16 hr and 10 minutes. Approximately one half of the reaction mixture was transferred from the reaction flask (flask 1) to a 50 L flask (flask 2) fitted with a mechanical stirrer. Then 27 L of deionized water was added to flask 1 and the mixture was stirrer for 10 minutes. The pH was taken using a pH meter; the pH was 11.56. The pH of the mixture was adjusted to 12.02 by the addition of 100 ml of the 30% sodium, methoxide solution in methanol; this gave a clear solution, (the reaction mixture at this time contained a small amount of solid. The pH was adjusted to give a clear solution from which the precipitation the product would be precipitated). The reaction mixture in flask 2 was diluted in the same manner, 27 L of deionized water was added, and the pH adjusted by the addition of 100 ml of a 30 % sodium methoxide solution to the mixture, to give a pH of 12.01 (clear solution).

The reaction mixture in each flask was acidified by the addition of glacial acetic acid to precipitate the product. Flask 1 had a final pH of 8.98, and Flask 2 had a final pH of 8.70. The product from both flasks was isolated by filtration using a Buchner funnel and filter cloth. The filter cake was washed with 15 L of deionized water, and the funnel was covered and the product was partially dried on the funnel under vacuum for 15.5 hr. The product was removed and placed into five glass trays. The trays were placed in a vacuum oven and the product was dried to constant weight. The first drying period was for 22 hours at 60°C using a Nash pump as the vacuum source with an argon bleed. The trays were removed from the vacuum oven and weighed. The trays were returned to the oven and the product dried for an additional 4 hr and 10 minutes using an oil pump as the vacuum source and with no argon bleed. The material was packaged in double 4-mill polyethylene bags, and placed in a plastic outer container. The final weight after sampling was 2633.4 g (95.6%).

### Step 4 - Recrystallization of Crude SAHA

The crude SAHA was recrystallized from methanol/water. A 50 L flask with a mechanical stirrer, thermocouple, condenser, and inlet for inert atmosphere was charged with the crude SAHA to be crystallized (2,525.7 g), followed by 2,625 ml of deionized water and 15,755 ml of methanol. The material was heated to reflux to give a solution. Then 5,250 ml of deionized water was added to the reaction mixture. The heat was turned off, and the mixture was allowed to cool. When the mixture had cooled sufficiently so that the flask could be safely handled (28°C), the flask was removed from the heating mantle, and placed in a tub for use as a cooling bath. Ice/water was added to the tub to cool the mixture to -5°C. The mixture was held below that temperature for 2 hours. The product was isolated by filtration, and the filter cake washed with 1.5 L of cold methanol/water (2:1). The funnel was covered, and the product was partially dried under vacuum for 1.75 hr. The product was removed from the funnel and placed in 6 glass trays. The trays were placed in a vacuum oven, and the product was dried for 64.75 hr at 60°C using a Nash pump as the vacuum source, and using an argon bleed. The trays were removed for weighing, and then returned to the oven and dried for an additional 4 hours at 60°C to give a constant weight. The vacuum source for the second drying period was an oil pump, and no argon bleed was used. The material was packaged in double 4-mill polyethylene bags, and placed in a plastic outer container. The final weight after sampling was 2,540.9 g (92.5%).

### EXAMPLE 2

### Oral dosing of suberoylanilide hydroxamic acid (SAHA)

Background: Treatment with hybrid polar cellular differentiation agents has resulted in the inhibition of growth of human solid tumor derived cell lines and xenografts. The effect is mediated in part by inhibition of histone deacetylase. SAHA is a potent histone deacetylase inhibitor that has been shown to have the ability to induce tumor cell growth arrest, differentiation, and apoptosis in the laboratory and in preclinical studies.

Objectives: To define a safe daily oral regimen of SAHA that can be used in Phase II studies. In addition, the pharmacokinetic profile of the oral formulation of SAHA was be evaluated. The oral bioavailability of SAHA in humans in the fasting vs. non-fasting state and anti-tumor effects of treatment were also monitored. Additionally, the biological effects of SAHA on normal tissues and tumor cells were assessed and responses with respect to levels of histone acetylation were documented.

Patients: Patients with histologically documented advanced stage, primary or metastatic adult solid tumors that are refractory to standard therapy or for which no curative standard therapy exists. Patients must have a Karnofsky Performance Status of ≥70%, and adequate hematologic, hepatic, and renal function. Patients must be at least four weeks from any prior chemotherapy, radiation therapy or other investigational anticancer drugs.

Dosing Schedule: On the first day, patients were first treated with 200 mg of intravenously-administered SAHA. Starting on the second day, patients were treated with daily doses of oral SAHA according to Table 1. Each cohort received a different dose of SAHA. "QD" indicates dosing once a day; "Q12 hours" indicates dosing twice a day. For example, patients in Cohort IV received two 800 mg doses of SAHA per day. Doses were administered to patients daily and continuously. Blood samples were taken on day one and on day 21 of oral treatment. Patients were taken off oral SAHA treatment due to disease progression, tumor regression, unacceptable side effects, or treatment with other therapies.

**Table 1: Oral SAHA Dose Schedule**

| Cohort | Oral Dose (mg) | Number of Days | Daily Dosing Schedule |
|---|---|---|---|
| I | 200 | Continuous | QD |
| II | 400 | Continuous | QD |
| III | 400 | Continuous | Q12 hours |
| IV | 800 | Continuous | Q12 hours |
| V | 1200 | Continuous | Q12 hours |
| VI | 1600 | Continuous | Q12 hours |
| VII | 2000 | Continuous | Q12 hours |

Results: Comparison of serum plasma levels shows high bioavailability of SAHA administered orally, both when the patient fasted and when the patient did not fast, compared to SAHA administered intravenously (IV SAGA). "AUC" is an estimate of the bioavailability of SAHA in (ng/ml)min, where 660 ng/ml is equal to 2.5 µM SAHA. The AUC taken together with the half-life (t½) shows that the overall bioavailability of oral SAHA is better than that of IV SAHA. Cₘₐₓ is the maximum concentration of SAHA observed after administration. IV SAHA was administered at 200 mg infused over two hours. The oral SAHA was administered in a single capsule at 200 mg. Tables 2 and 3 summarize the results of an HPLC assay (LCMS using a deuterated standard) that quantitates the amount of SAHA in the blood plasma of the patients versus time; using acetylated histone-4 (α-AcH4) as a marker.

**Table 2: Serum Plasma Levels of Oral SAHA - Patient #1**

| | IV | Oral (fasting) | Oral (nonfasting) |
|---|---|---|---|
| Cₘₐₓ (ng/ml) | 1329 | 225 | 328 |
| t_{½} (min) | 20 | 80 | 64 |
| AUC (ng/ml)min | 153,000 | 25,000 | 59,000 |

**Table 3: Serum Plasma Levels of Oral SAHA - Patient #2**

| | IV | Oral (fasting) | Oral (nonfasting) |
|---|---|---|---|
| Cₘₐₓ (ng/ml) | 1003 | 362 | 302 |
| t_{½} (min) | 21 | 82 | 93 |
| AUC (ng/ml)min | 108,130 | 63,114 | 59,874 |

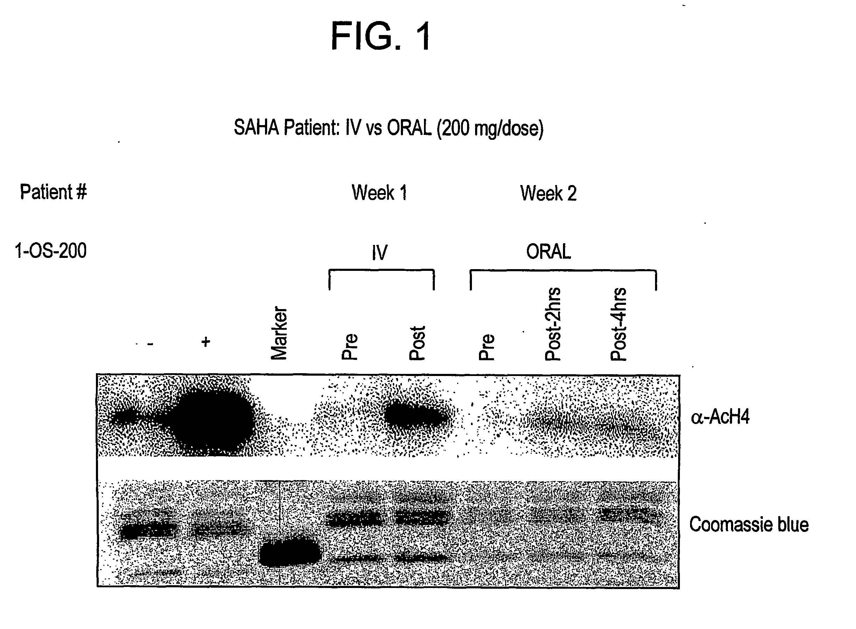

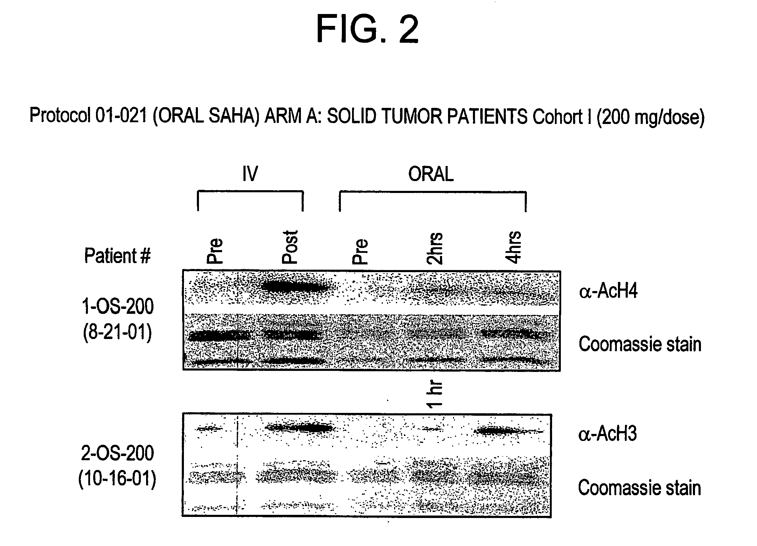

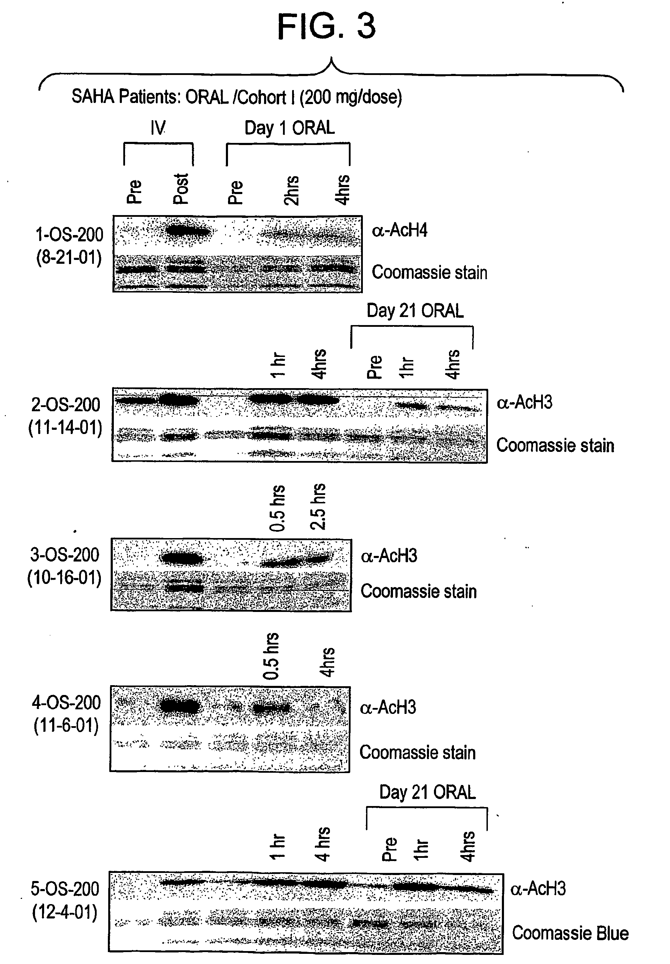

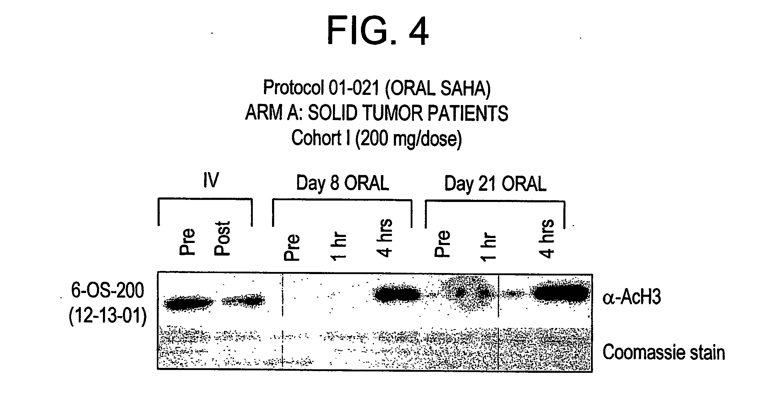

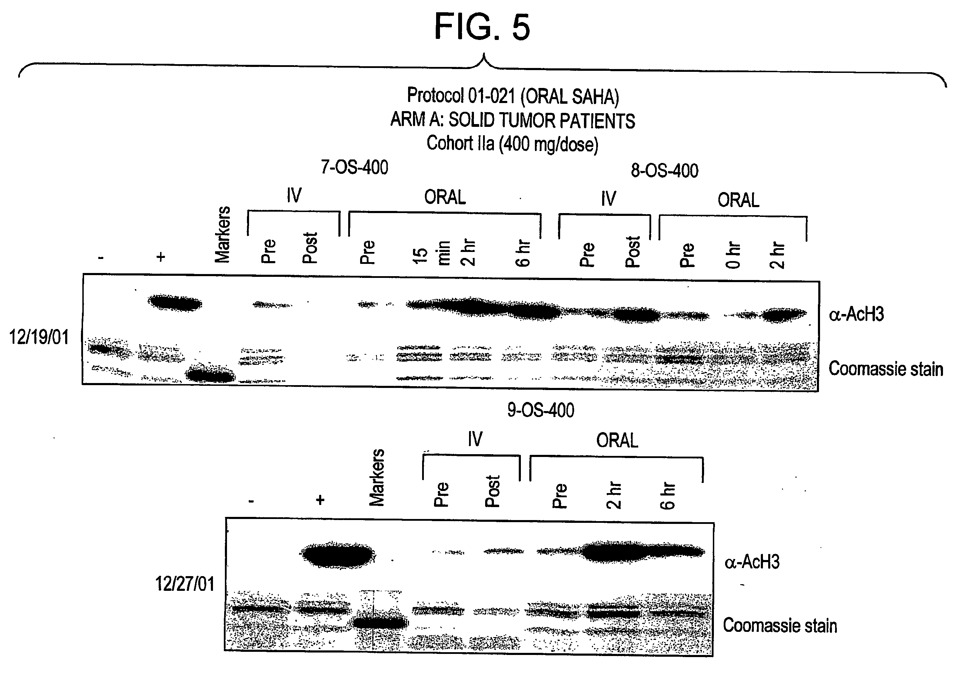

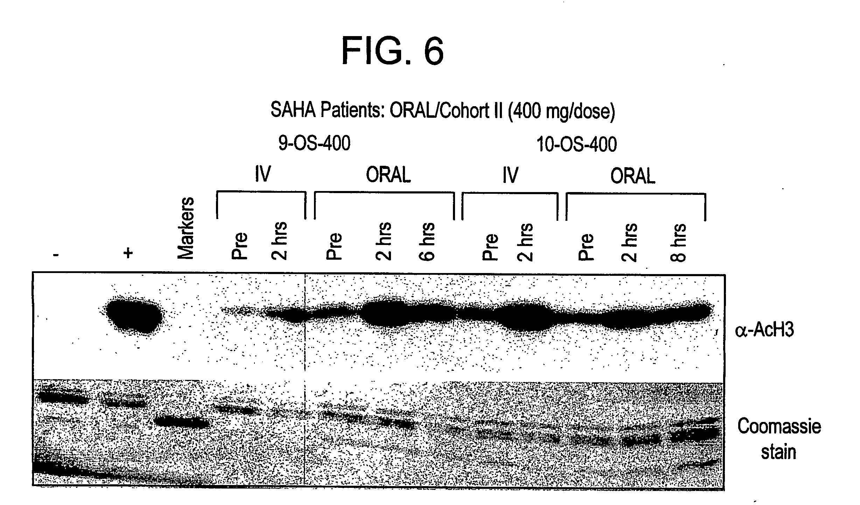

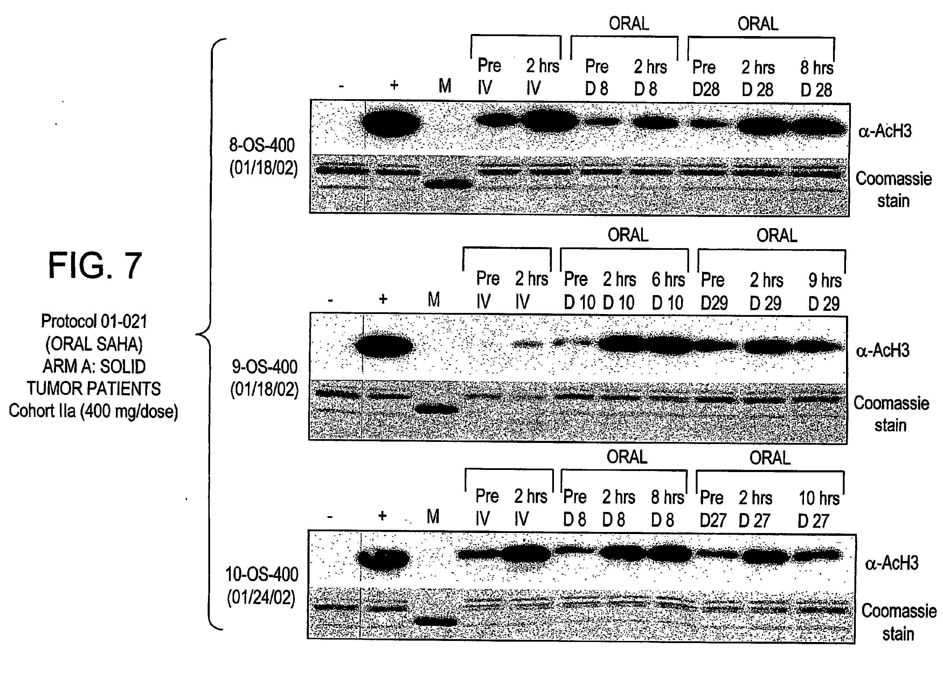

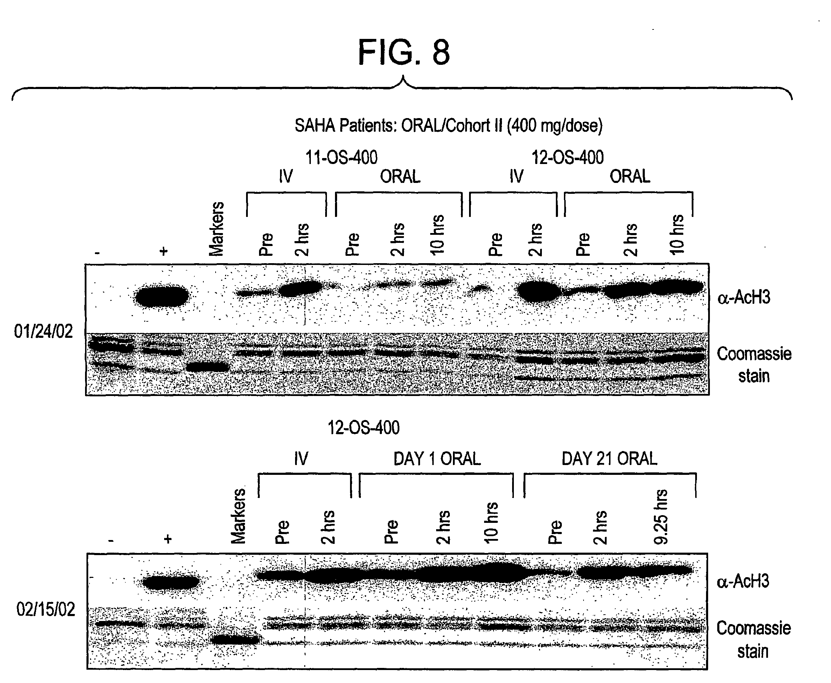

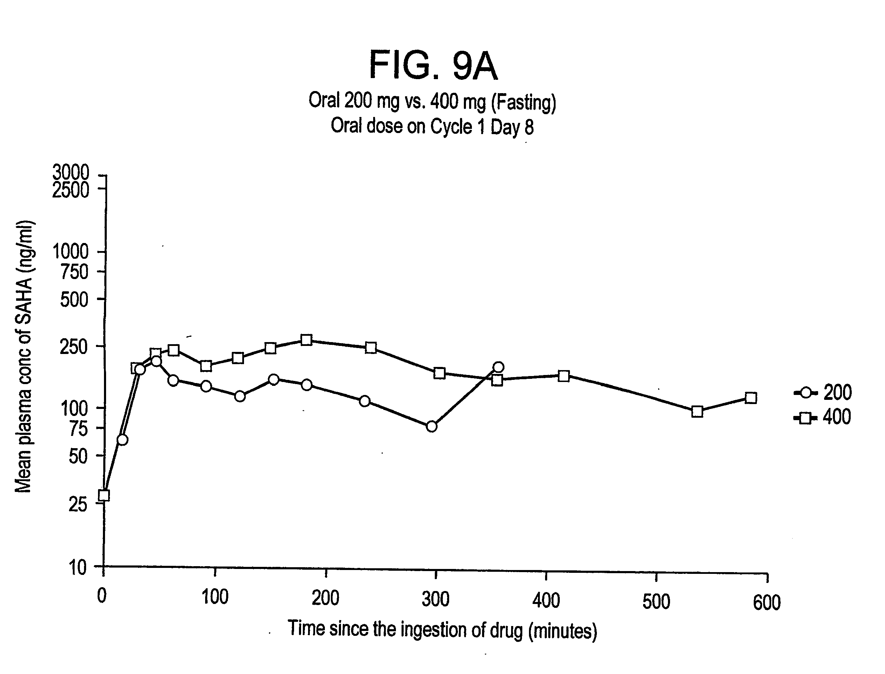

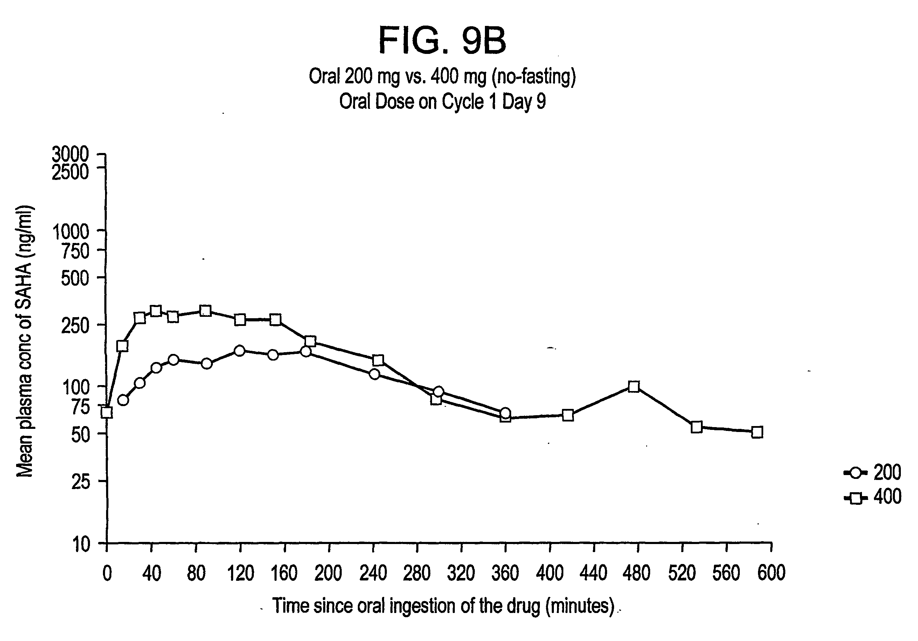

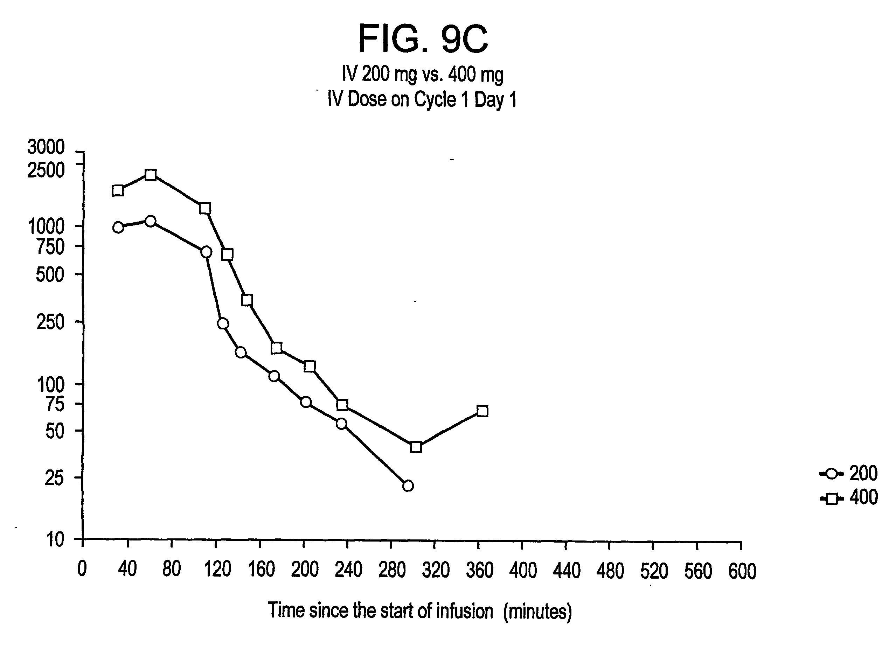

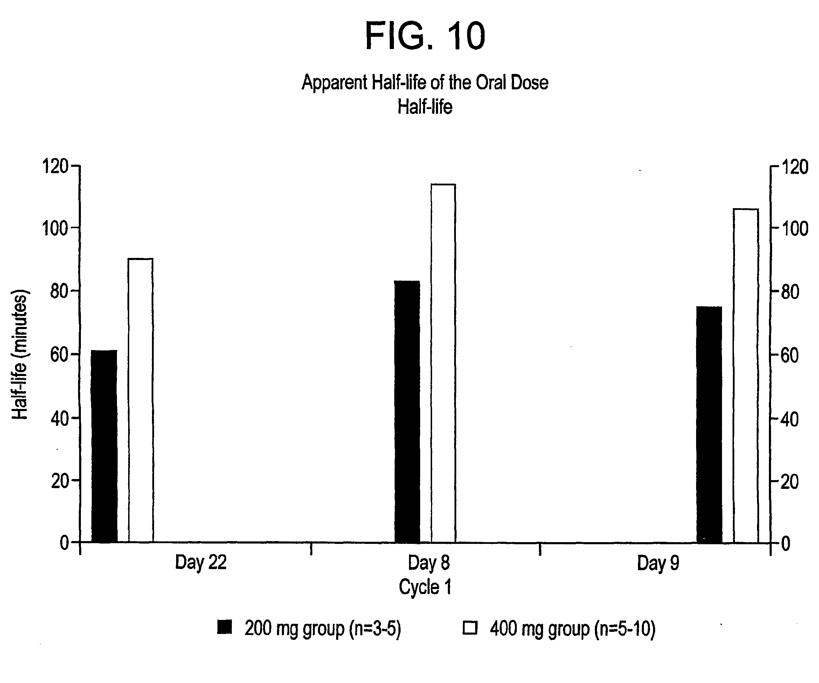

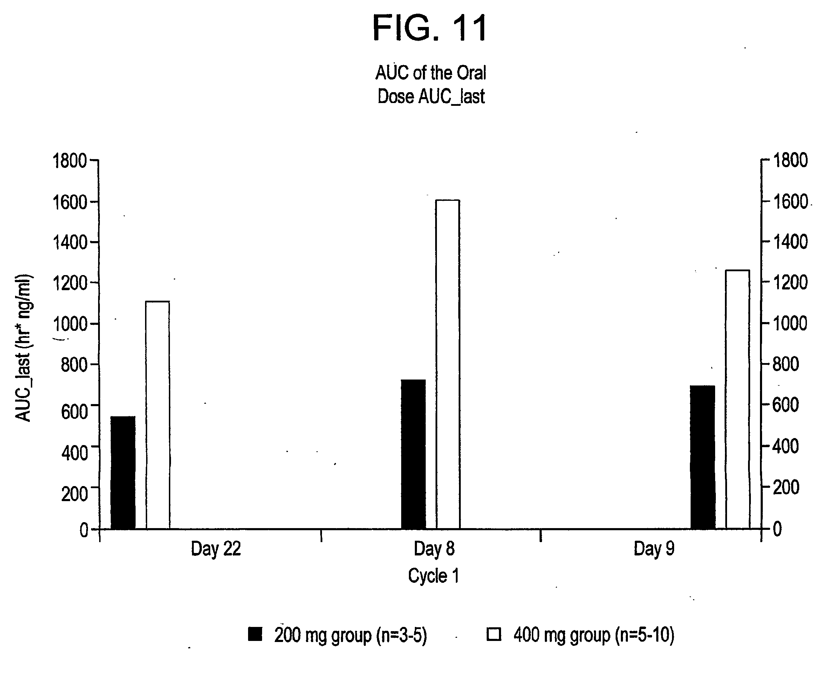

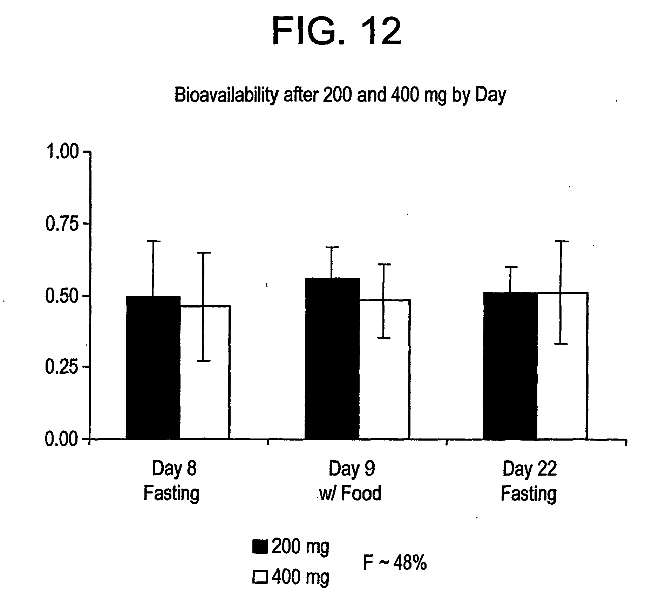

Figures 1 to 8 are HPLC slides showing the amount of α-AcH4 in patients in Cohorts I and II, measured at up to 10 hours after receiving the oral dose, compared with the α-AcH4 levels when SAHA was administered intravenously. Fig 9 shows the mean plasma concentration of SAHA (ng/ml) at the indicated time points following administration. Fig 9A: Oral dose (200 mg and 400 mg) under fasting on Day 8. Fig 9B: Oral dose (200 mg and 400 mg) with food on Day 9. Fig 9C: IV dose on day 1. Fig 10 shows the apparent half-life of a SAHA 200 mg and 400 mg oral dose, on Days 8, 9 and 22. Fig 11 shows the AUC (ng/ml/hr) of a SAHA 200 mg and 400 mg oral dose, on Days 8, 9 and 22. Figure 12 shows the bioavailability of SAHA after a 200 mg and 400 mg oral dose, on Days 8, 9 and 22.

### EXAMPLE 3

### Oral dosing of suberoylanilide hydroxamic acid (SAHA) - Dose Escalation

In another experiment, twenty-five patients with solid tumors have been enrolled onto arm A, thirteen patients with Hodgkin's or non-Hodgkin's lymphomas have been enrolled onto arm B, and one patient with acute leukemia and one patient with myelodysplastic syndrome have been enrolled onto arm C, as shown in Table 4.

**Table 4: Dose Escalation Scheme and Number of Patients on Each Dose Level**

| | Dose | Dosing | #Days of Dosing | Rest Period | #Patients Enrolled |
|---|---|---|---|---|---|
| Cohort | (mg/day) | Schedule | | | (arm A/arm B/arm C)* |
| I | 200 | Once a day | Continuous | None | 6/0/0 |
| II | 400 | Once a day | Continuous | None | 5/4/2 |
| III | 400 | q 12 hours | Continuous | None | 6/3/0 |
| IV | 600 | Once a day | Continuous | None | 4/3/0 |
| V | 200 | q 12 hours | Continuous | None | 4/3/0 |
| VI | 300 | q 12 hours | Continuous | None | -/-/- |
| | | | | | Sub-totals: 25/13/2 |
| | | | | | Total = 40 |

| | | | | | |
|---|---|---|---|---|---|
| *Arm A= solid tumor, arm B= lymphoma, arm C= leukemia | | | | | |

### Results:

Among eleven patients treated in Cohort II, one patient experienced the DLT of grade 3 diarrhea and grade 3 dehydration during the first treatment cycle. Nine patients were entered into Cohort III. Two patients were not evaluable for the 28-day toxicity assessment because of early study termination due to rapid progression of disease. Of the seven remaining patients, five experienced DLT during the first treatment cycle: diarrhea/dehydration (n=1), fatigue/dehydration (n=1), anorexia (n=1), dehydration (n=1) and anorexia/dehydration (n=1). These five patients recovered in approximately one week after the study drug was held. They were subsequently dose reduced to 400 mg QD, which appeared to be well tolerated. The median days on 400 mg BID for all patients in Cohort III was 21 days. Based on these findings the 400 mg q12 hour dosing schedule was judged to have exceeded the maximally tolerated dose. Following protocol amendment, accrual was continued in cohort IV at a dose of 600 mg once a day. Of the seven patients enrolled onto cohort IV, two were not evaluable for the 28-day toxicity assessment because of early study termination due to rapid progression of disease. Three patients experienced DLT during the first treatment cycle: anorexia/dehydration/fatigue (n=1), and diarrhea/dehydration (n=2). The 600 mg dose was therefore judged to have exceeded the maximally tolerated dose and the 400 mg once a day dose was defined as the maximally tolerated dose for once daily oral administration. The protocol was amended to evaluate additional dose levels of the twice a day dosing schedule at 200 mg BID and 300 mg BID administered continuously.

The interim pharmacokinetic analysis was based on 18 patients treated on the dose levels of 200 mg QD, 400 mg QD, and 400 mg BID. In general, the mean estimates of Cₘₐₓ and AUC_{inf} of SAHA administered orally under fasting condition or with food increased proportionally with dose in the 200 mg to 400 mg dose range. Overall, the fraction of AUC_{inf} due to extrapolation was 1% or less. Mean estimates for apparent half-life were variable across dose groups under fasting condition or with food, ranging from 61 to 114 minutes. The mean estimates of Cₘₐₓ, varies from 233 ng/ml (0.88 µM) to 570 ng/ml (2.3 µM). The bioavailable fraction of SAHA, calculated from the AUC_{inf} values after the IV infusion and oral routes, was found to be approximately 0.48.

Peripheral blood mononuclear cells were collected pre-therapy, immediately post-infusion and between 2 - 10 hours after oral ingestion of the SAHA capsules to assess the effect of SAHA on the extent of histone acetylation in a normal host cell. Histones were isolated and probed with anti-acetylated histone (H3) antibody followed by HRP-secondary antibody. Preliminary analysis demonstrated an increase in the accumulation of acetylated histones in peripheral mononuclear cells that could be detected up to 10 hours after ingestion of SAHA capsules at 400 mg per day dose level.

Thirteen patients continued treatment for 3-12 months with responding or stable disease: thyroid (n=3), sweat gland (n=1), renal (n=2), larynx (n=1), prostate (n=1), Hodgkin's lymphoma (n=2), non-Hodgkin's lymphoma (n=2), and leukemia (n=1).

Six patients had tumor shrinkage on CT scans. Three of these six patients meet the criteria of partial response (one patient with metastatic laryngeal cancer and two patients with non-Hodgkin's lymphomas). These partial responses occurred at the dose levels of 400 mg BID (n=2) and 600 mg QD (n=1).

The dosages described above have also been administered twice daily intermittently. Patients have received SAHA twice daily three to five days per week. Patient response has been seen with administration of SAHA twice daily at 300 mg for three days a week.

### EXAMPLE 4

### Intravenous Dosing of SAHA

Table 5 shows a dosing schedule for patients receiving SAHA intravenously. Patients begin in Cohort I, receiving 300 mg/m² of SAHA for five consecutive days in a week for one week, for a total dose of 1500 mg/m². Patients were then observed for a period of two weeks and continued to Cohort II, then progressed through the Cohorts unless treatment was terminated due to disease progression, tumor regression, unacceptable side effects or the patient received other treatment.

**Table 5: Standard Dose Escalation for Intravenously-Administered SAHA**

| Cohort | Dose | Number of Days/Week | Number of Consecutive Weeks | Observation Period | Total Dose |
|---|---|---|---|---|---|
| | (mg/m²) | | | (Weeks) | (mg/m²) |
| I | 300 | 5 | 1 | 2 | 1500 |
| II | 300 | 5 | 2 | 2 | 3000 |
| III | 300 | 5 | 3 | 1* | 4500 |
| IV | 600 | 5 | 3 | 1* | 9000 |
| V | 800 | 5 | 3 | 1* | 13500 |
| VI | 1200 | 5 | 3 | 1* | 18000 |
| VII | 1500 | 5 | 3 | 1* | 22500 |

| | | | | | |
|---|---|---|---|---|---|
| *Hematologic patients started at dose level III. | | | | | |

### EXAMPLE 5

### Treatment of Mesothelioma with SAHA

Three patients with mesothelioma were enrolled in Phase I studies with SAHA. The patients were administered oral SAHA twice daily at a dose of 300 mg or 400 mg for three days a week. One partial response was observed following SAHA treatment according to the above regimen for 6 months.

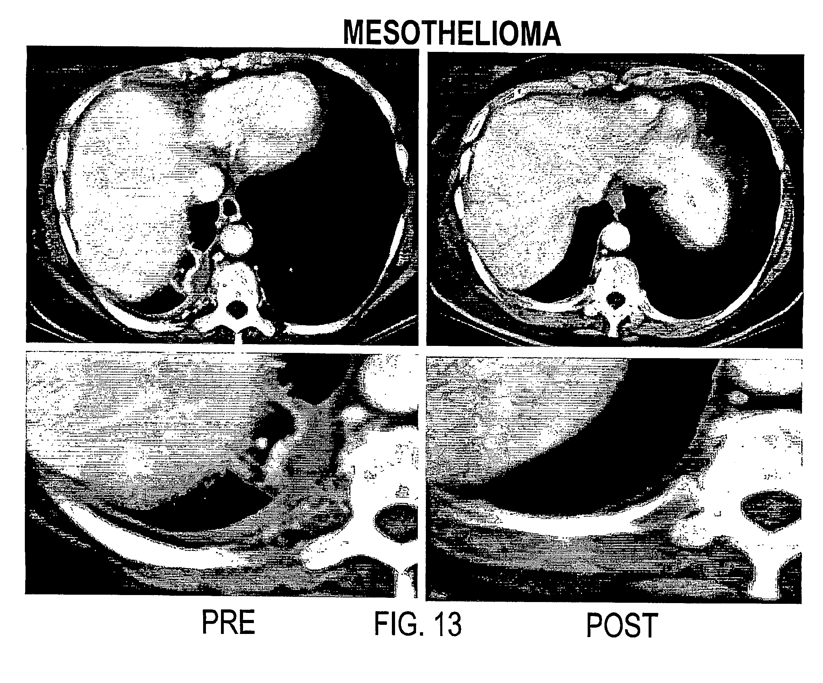

Figure 13 is a CT scan of a mesothelioma tumor from a patient, before (PRE - left panel) and after (POST - right panel) treatment with SAHA twice daily at a dose of 300 mg three days a week for 6 months. The data demonstrates that SAHA is effective at treating mesothelioma tumors in patients.

While this invention has been particularly shown and described with references to preferred embodiments thereof, it will be understood by those skilled in the art that various changes in form and details may be made therein without departing from the meaning of the invention described The scope of the invention includes the subject matter of the claims that follow.

### References

1. Spoin, M. B., Roberts, A. B., and Driscoll, J. S. (1985) in Cancer: Principles and Practice of Oncology, eds. Hellman, S., et al., Ed. 2, (J. B. Lippincott, Philadelphia), P. 49.
2. Breitman, T. R, Selonick, S. E., and Collins, S. J. (1980) Proc. Natl. Acad. Sci. USA 77; 2936-2940.
3. Olsson, L L. and Breitman, T. R. (1982) Cancer Res. 42: 3924-3927.
4. Schwartz, E. L. and Sartorelli, A. C. (1982) Cancer Res. 42: 2651-2655.
5. Marks, P. A., Sheffery, M., and Rifkind, R. A. (1987) Cancer Res. 47: 659.
6. Sachs, L. (1978) Nature (Lond.) 274: 535.
7: Friend, C:, Scher, W., Holland, J. W., and Sato, T. (1971) Proc. Natl. Acad. Sci. (USA) 68: 378-382.
8. Tanaka, M., Levy, J., Terada, M., Breslow, R., Rifkind, R. A., and Marks, P. A. (1975) Proc. Natl. Acad. Sci. (USA) 72: 1003-1006.
9. Reuben, R. C., Wife, R. L., Breslow, R., Rifkind, R. A., and Marks, P. A. (1976) Proc. Natl. Acad. Sci. (USA) 73: 862-866.
10. Abe, E., Miyaura, C., Sakagami, H., Takeda, M., Konno, K., Yamazaki, T., Yoshika, S., and Suda, T. (1981) Proc. Natl. Acad. Sci, (USA) 78: 4990-4994.
11. Schwartz, E. L., Snoddy, J. R., Kreutter, D., Rasmussen, H., and Sartorelli, A. C. (1983) Proc. Am. Assoc. Cancer Res. 24: 18.
12. Tanenaga, K., Hozumi, M., and Sakagami, Y. (1980) Cancer Res. 40: 914-919.
13. Lotem, J. and Sachs, L. (1975) Int. J. Cancer 15: 731-740.
14. Metcalf, D. (1985) Science, 229: 16-22.
15. Scher, W., Scher, B. M., and Waxman, S. (1983) Exp. Hematol. 11: 490-498.
16. Scher, W., Scher, B. M., and Waxman, S. (1982) Biochem. & Biophys. Res. Comm. 109: 348-354.
17. Huberman, E. and Callaham, M. F. (1979) Proc. Natl. Acad. Sci. (USA) 76: 1293-1297.
18. Lottem, J. and Sachs, L. (1979) Proc. Natl. Acad. Sci. (USA) 76: 5158-5162.
19. Terada, M., Epner, E., Nudel, U., Salmon, J., Fibach, E., Rifkind, R. A., and Marks, P. A. (1978) Proc. Natl. Acad. Sci. (USA) 75: 2795-2799.
20. Morin, M. J. and Sartorelli, A. C. (1984) Cancer Res. 44: 2807-2812.
21. Schwartz, E. L., Brown, B. J., Nierenberg, M., Marsh, J. C., and Sartorelli, A. C. (1983) Cancer Res. 43: 2725-2730.
22. Sugano, H., Furusawa, M., Kawaguchi, T., and Ikawa, Y. (1973) Bibl. Hematol. 39: 943-954.
23. Ebert, P. S., Wars, I., and Buell, D. N. (1976) Cancer Res. 36: 1809-1813.
24. Hayashi, M., Okabe, J., and Hozumi, M. (1979) Gann 70: 235-238.
25. Fibach, E., Reuben, R. C., Rifkind, R. A., and Marks, P. A. (1977) Cancer Res. 37: 440-444.
26. Melloni, E., Pontremoli, S., Damiani, G., Viotti, P., Weich, N., Rifkind, R. A., and Marks, P. A. (1988) Proc. Natl. Acad. Sci. (USA) 85: 3835-3839.
27. Reuben, R., Khanna, P. L., Gazitt, Y., Breslow, R., Rifkind, R. A., and Marks, P. A. (1978) J. Biol. Chem. 253: 4214-4218.
28. Marks, P. A. and Rifkind, R A. (1988) Int. Journal of Cell Cloning 6:230-240.
29. Melloni, E., Pontremoli, S., Michetti, M., Sacco, O., Cakiroglu, A. G., Jackson, J. F., Rifkind, R. A., and Marks, P. A. (1987) Proc. Natl. Acad. Sciences (USA) 84: 5282-5286.
30. Marks, P. A. and Rifkind, R. A. (1984) Cancer 54: 2766-2769.
31. Egorin, M. J., Sigman, L. M. VanEcho, D. A., Forrest, A., Whitacre, M. Y., and Aisner, J. (1987) Cancer. Res. 47: 617-623.
32. Rowinsky, E. W., Ettinger, D. S., Grochow, L. B., Brundrett, R. B., Cates, A. E., and Donehower, R. C. (1986) J. Clin. Oncol. 4: 1835-1844.
33. Rowinsky, E. L. Ettinger, D. S., McGuire, W. P., Noe, D. A., Grochow, L. B., and Donehower, R. C. (1987) Cancer Res. 47: 5788-5795.
34. Callery, P. S., Egorin, M. J., Geelhaar, L. A., and Nayer, M. S. B. (1986) Cancer Res. 46: 4900-4903.
35. Young, C. W. Fanucchi, M. P., Walsh, T. B., Blatzer, L., Yaldaie, S., Stevens, Y. W., Gordon, C., Tong, W., Rifkind, R. A., and Marks, P. A. (1988) Cancer Res. 48: 7304-7309.
36. Andreeff, M., Young, C., Clarkson, B., Fetten, J., Rifkind, R. A., and Marks, P. A. (1988) Blood 72: 186a.
37. Marks, P. A., Breslow, R., Rifkind, R. A., Ngo, L., and Singh, R. (1989) Proc. Natl. Acad. Sci. (USA) 86: 6358-6362.
38. Breslow, R., Jursic, B., Yan, Z. F., Friedman, E., Leng, L., Ngo, L., Rifkind, R. A., and Marks, P. A. (1991) Proc. Natl. Acad. Sci. (USA) 88: 5542-5546.
39. Richon, V.M., Webb, Y., Merger, R., et al. (1996) PNAS 93:5705-8.
40. Cohen, L.A., Amin, S., Marks, P.A., Rifkind, R.A., Desai, D., and Richon, V.M. (1999) Anticancer Research 19:4999-5006.
41. Grunstein, M. (1997) Nature 389:349-52.
42. Finnin, M.S., Donigian, J.R., Cohen, A., et al. (1999) Nature 401:188-193.
43. Van Lint, C., Emiliani, S., Verdin, E. (1996) Gene Expression 5:245-53.
44. Archer, S. Shufen, M. Shei, A., Hodin, R. (1998) PNAS 95:6791-96.
45. Dressel, U., et al. (2000) Anticancer Research 20(2A):1017-22.
46. Lin, R.J., Nagy, L., Inoue, S., et al. (1998) Nature 391:811-14.

## Claims

1. Use of suberoylanilide hydroxamic acid (SAHA) represented by the structure: 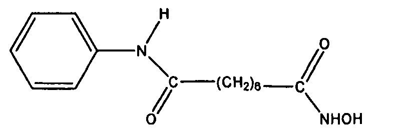 or a pharmaceutically acceptable salt or hydrate thereof, and a pharmaceutically acceptable carrier or diluent, for the preparation of an oral medicament for administration to a subject for treatment of mesothelioma.

2. The use of claim 1, wherein said medicament is for administration twice daily at a dose of 300 mg intermittently.

3. The use of claim 2, wherein said medicament is for administration three to five days per week.

4. The use of claim 2, wherein said medicament is for administration three days a week.

5. The use of claim 1, wherein SAHA is the active ingredient in said medicament.

## Patentansprüche

1. Verwendung von Suberoylanilid-hydroxamsäure (SAHA), die repräsentiert wird durch die Struktur: 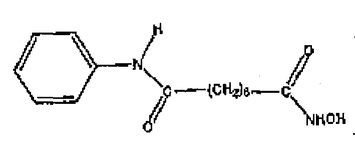 oder eines pharmazeutisch annehmbaren Salzes oder Hydrats davon, und eines pharmazeutisch annehmbaren Trägers oder Verdünnungsmittels, zur Herstellung eines oralen Arzneimittels zur Verabreichung an ein Individuum zur Behandlung von Mesotheliomen.

2. Verwendung nach Anspruch 1, wobei das Arzneimittel zur Verabreichung zweimal täglich mit einer Dosis von 300mg, mit Unterbrechungen, ist.

3. Verwendung nach Anspruch 2, wobei das Arzneimittel zur Verabreichung 3 bis 5 Tage pro Woche ist.

4. Verwendung nach Anspruch 2, wobei das Arzneimittel zur Verabreichung an 3 Tagen einer Woche ist.

5. Verwendung nach Anspruch 1, wobei SAHA der Wirkstoff in dem Arzneimittel ist.

## Revendications

1. Utilisation de l'acide subéroylanilide hydroxamique (SAHA) représenté par la structure : 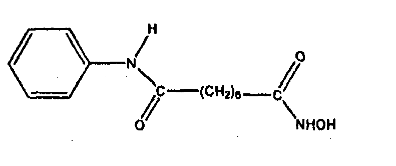 ou d'un sel pharmaceutiquement acceptable de celui-ci ou d'un hydrate de celui-ci, et d'un support ou diluant pharmaceutiquement acceptable, pour la préparation d'un médicament pour voie orale, pour administration à un sujet en vue du traitement d'un mésothéliome.

2. Utilisation de la revendication 1, dans laquelle ledit médicament est destiné à une administration à raison de deux fois par jour à une dose de 300 mg par intermittence.

3. Utilisation de la revendication 2, dans laquelle ledit médicament est destiné à une administration à raison de trois à cinq jours par semaine.

4. Utilisation de la revendication 2, dans laquelle ledit médicament est destiné à une administration à raison de trois jours par semaine.

5. Utilisation de la revendication 1, dans laquelle le SAHA est le principe actif dans ledit médicament.
